# EUROPÄISCHE PATENTSCHRIFT

(11) **EP 0 431 291 B1**
(45) Veröffentlichungstag und Bekanntmachung des Hinweises auf die Patenterteilung: **29.07.1998**
(21) Anmeldenummer: 90120153.3
(22) Anmeldetag: 20.10.1990
(51) Int. Cl.: C07D 249/12, C07D 409/12, C07D 401/12, C07D 403/12, C07D 417/12, C07D 401/14, C07D 413/12, A01N 47/38

(54) **Sulfonylaminocarbonyltriazolinone mit über Schwefel gebundenen Substituenten**
Sulfonylaminocarbony-triazolinones having substituents attached through sulfur
Sulfonylaminocarbonyl-triazolinones comportant des substituants attachés par du soufre

(30) Priorität: 03.11.1989 DE 3936623
(43) Veröffentlichungstag der Anmeldung: 12.06.1991
(73) Patentinhaber: BAYER AG, 51368 Leverkusen (DE)
(72) Erfinder: Müller, Klaus-Helmut, Dr., W-4000 Düsseldorf 13 (DE); Babczinski, Peter, Dr., W-5600 Wuppertal (DE); Santel, Hans-Joachim, Dr., W-5090 Leverkusen 1 (DE); Schmidt, Robert R., Dr., W-5060 Bergisch-Gladbach 2 (DE)

(56) Entgegenhaltungen:
- EP-A- 0 283 876
- EP-A- 0 341 489
- EP-A- 0 391 187

## Beschreibung

Die Erfindung betrifft neue Sulfonylaminocarbonyltriazolinone mit über Schwefel gebundenen Substituenten, mehrere Verfahren zu ihrer Herstellung und ihre Verwendung als Herbizide.

Es ist bekannt, daß bestimmte substituierte Aminocarbonylimidazolidinone, wie z. B. 1-Isobutylaminocarbonyl-2-imidazolidinon (Isocarbamid), herbizide Eigenschaften aufweisen (vgl, R. Wegler, Chemie der Pflanzenschutzund Schadlingsbekampfungsmittel, Band 5, S. 219, Springer-Verlag, Berlin-Heidelberg-New York, 1977).

Es ist weiterhin bekannt, daß auch bestimmte Aminocarbonyltriazolinone mit über Schwefel gebundenen Substituenten (aber ohne N-Sulfonylgruppen) herbizide Eigenschaften besitzen (vgl. EP-A-283 876).

Die Wirkung dieser vorbekannten Verbindungen ist jedoch nicht in allen Belangen zufriedenstellend.

Ferner werden in einer prioritätsälteren, aber nachveröffentlichten Patentanmeldung (vgl. EP-A-341 489) substituierte Sulfonylaminocarbonyltriazolinone und deren Verwendung als Herbizide beschrieben, wobei unter anderem und in ganz allgemeiner Form auch solche Triazolinone mit über Schwefel gebundenen Substituenten (nämlich 5-Alkylthio-Resten) vorgeschlagen werden; die Beschreibung enthält jedoch kein konkretes Beispiel für diese dort erstmals vorgeschlagene Stoffgruppe.

Es wurden nun die neuen Sulfonylaminocarbonyl-triazolinone mit über Schwefel gebundenen Substituenten der allgemeinen Formel (I) 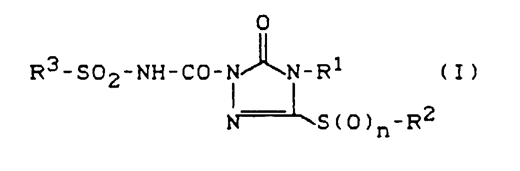 gefunden, in welcher
- n: für die Zahlen 0, 1 oder 2 steht,
- R¹: für gegebenenfalls durch Fluor, Chlor oder Brom substituiertes Methyl, für gegebenenfalls durch Fluor, Chlor und/oder Brom substituiertes Allyl, für gegebenenfalls durch Fluor, Chlor, Brom und/oder Methyl substituiertes Cyclopropyl, für gegebenenfalls durch Fluor, Chlor oder Cyano substituiertes C₁-C₆-Alkoxy oder für C₃-C₄-Alkenyloxy steht,
- R²: für gegebenenfalls durch Fluor, Chlor, Cyano, Cyclopropyl oder C₁-C₄-Alkoxy substituiertes C₁-C₆-Alkyl, für jeweils gegebenenfalls durch Fluor, Chlor und/oder Brom substituiertes C₃-C₆-Alkenyl oder C₃-C₆-Alkinyl oder für gegebenenfalls durch Fluor, Chlor, Brom und/oder C₁-C₄-Alkyl substituiertes C₃-C₆-Cycloalkyl steht, mit der Maßgabe, daß R² nicht für jeweils unsubstituiertes Methyl oder Ethyl steht, wenn R¹ für unsubstituiertes Methyl und n für O steht, und
- R³: für die Gruppierung 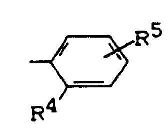 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxycarbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxycarbonyl substituiert ist) steht,
- R⁴ und/oder R⁵: weiterhin für Phenyl oder Phenoxy, für C₁-C₄-Alkylcarbonylamino, C₁-C₄-Alkoxycarbonylamino, C₁-C₄-Alkylamino-carbonyl-amino, Di-(C₁-C₄-alkyl)-amino-carbonylamino, oder für den Rest -CO-R⁸ stehen, wobei
R⁸ für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
- R⁴ und/oder R⁵: weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht, weiterhin
- R³: für den Rest 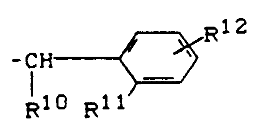 steht, worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen; weiterhin
- R³: für den Rest 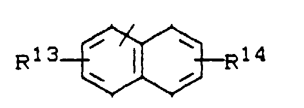 steht, worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen; weiterhin
- R³: für den Rest 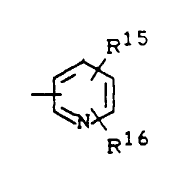 steht, worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxycarbonyl oder Dimethylaminocarbonyl stehen; weiterhin
- R³: für den Rest 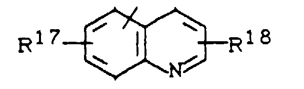 steht, worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)aminosulfonyl stehen; weiterhin
- R³: für den Rest 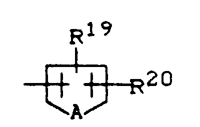 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht; weiterhin
- R³: für den Rest 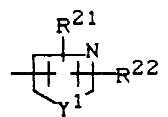 steht, worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
- R³: für den Rest 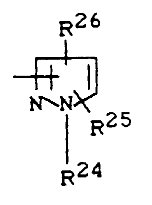 steht, worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht; weiterhin
- R³: für eine der nachstehend aufgeführten Gruppierungen steht, 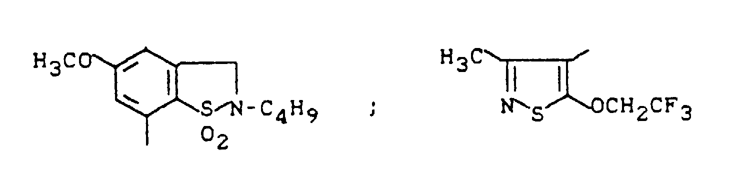 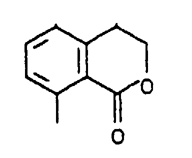 sowie Salze von Verbindungen der Formel (I).

Man erhält die neuen Sulfonylaminocarbonyltriazolinone mit über Schwefel gebundenen Substituenten der allgemeinen Formel (I) bzw, deren Salze, wenn man
a) Triazolinone der allgemeinen Formel (II) 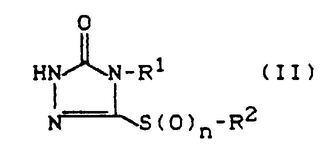 in welcher
   - n, R¹ und R²: die oben angegebenen Bedeutungen haben, mit Sulfonylisocyanaten der allgemeinen Formel (III)

   R³-SO₂-N=C=O (III)

   in welcher
   - R³: die oben angegebene Bedeutung hat, gegebenenfalls in Gegenwart eines Verdünnungsmittels umsetzt, oder wenn man
b) Triazolinon-Derivate der allgemeinen Formel (IV) 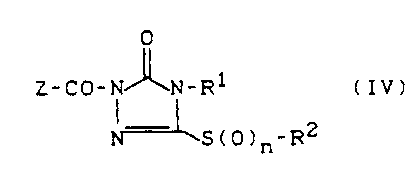 in welcher
   - n, R¹ und R²: die oben angegebenen Bedeutungen haben und
   - Z: für Chlor, C₁-C₄-Alkoxy, Benzyloxy oder Phenoxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt, oder wenn man
c) Triazolinone der allgemeinen Formel (II) 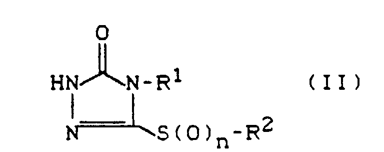 in welcher
   - n, R¹ und R²: die oben angegebenen Bedeutungen haben,
   mit Sulfonsäureamid-Derivaten der allgemeinen Formel (VI)

   R³-SO₂-NH-CO-Z (VI)

   in welcher
   - R³: die oben angegebene Bedeutung hat und
   - Z: für Chlor, C₁-C₄-Alkoxy, Benzyloxy oder Phenoxy steht,
   gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

Die neuen Sulfonylaminocarbonyltriazolinone mit über Schwefel gebundenen Substituenten der allgemeinen Formel (I) und ihre Salze zeichnen sich durch starke herbizide Wirksamkeit aus.

Überraschenderweise zeigen die neuen Verbindungen der Formel (I) erheblich bessere herbizide Wirkung als das strukturell ähnliche bekannte Herbizid 1-Isobutylaminocarbonyl-2-imidazolidinon (Isocarbamid).

Gegenstand der Erfindung sind vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, C₅- oder C₆-Cycloalkyl-ammonium- und Di-(C₁-C₂-alkyl)-benzyl-ammonium-Salze von Verbindungen der Formel (I), in welcher n, R¹, R² und R³ die oben angegebenen Bedeutungen haben.

Gegenstand der Erfindung sind weiter vorzugsweise Verbindungen der Formel (I), in welcher
- n: für die Zahlen 0, 1 oder 2 steht,
- R¹: für gegebenenfalls durch Fluor substituiertes Methyl, für Allyl, für Cyclopropyl, für C₁-C₄-Alkoxy oder für C₃-C₄-Alkenyloxy steht,
- R²: für gegebenenfalls durch Fluor, Chlor, Methoxy oder Ethoxy substituiertes C₁-C₄-Alkyl, für gegebenenfalls durch Fluor und/oder Chlor substituiertes C₃-C₄-Alkenyl, oder für C₃-C₆-Cycloalkyl steht,
mit der Maßgabe, daß R² nicht für jeweils unsubstituiertes Methyl oder Ethyl steht, wenn R¹ für unsubstituiertes Methyl und n für O steht, und
- R³: für die Gruppierung 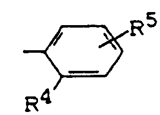 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Methoxyaminosulfonyl, Phenyl, Phenoxy oder C₁-C₃-Alkoxy-carbonyl steht und
R⁵ für Wasserstoff, Fluor, Chlor oder Brom steht; weiterhin
- R³: für den Rest 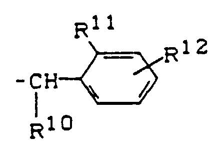 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht; weiterhin
- R³: für den Rest 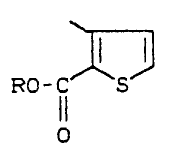 steht,
worin R für C₁-C₄-Alkyl steht, oder
- R³: für den Rest 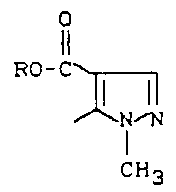 steht,
worin R für C₁-C₄-Alkyl steht.

Beispiele für die erfindungsgemäßen Verbindungen sind in der nachstehenden Tabelle 1 aufgeführt - vgl. auch die Herstellungsbeispiele.

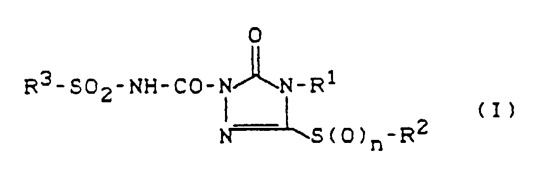

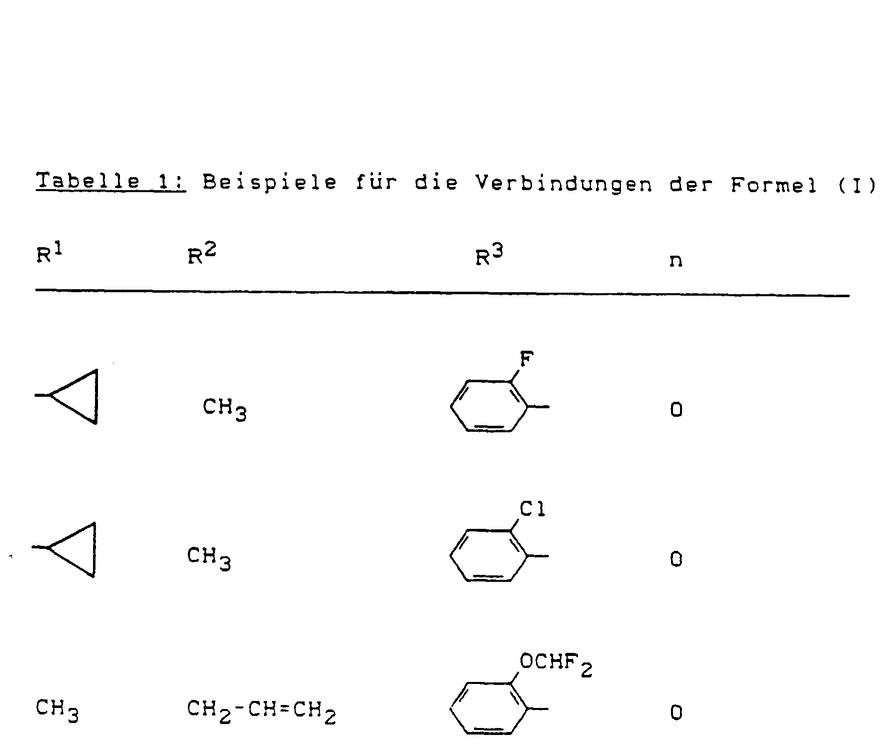

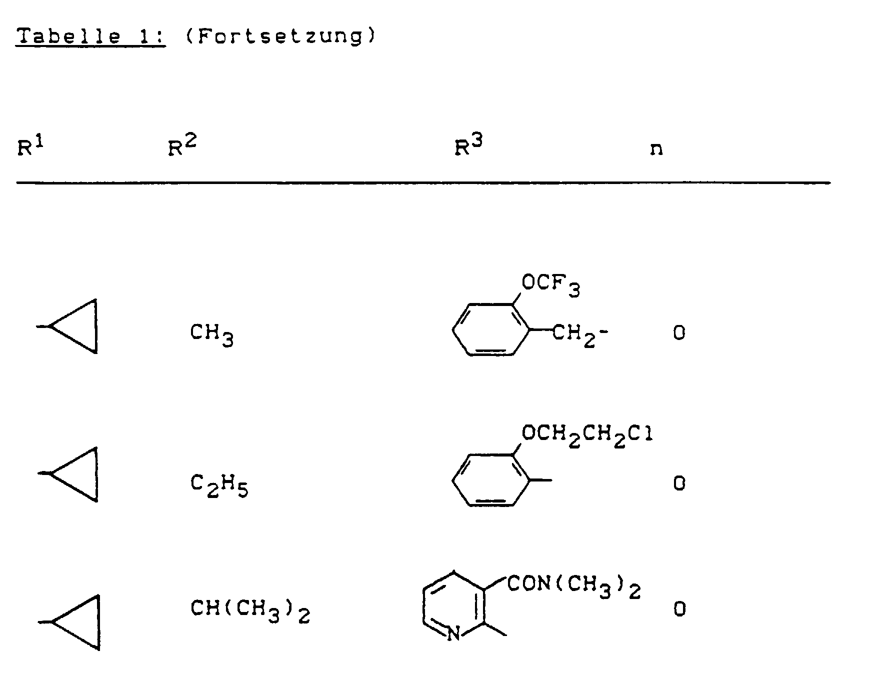

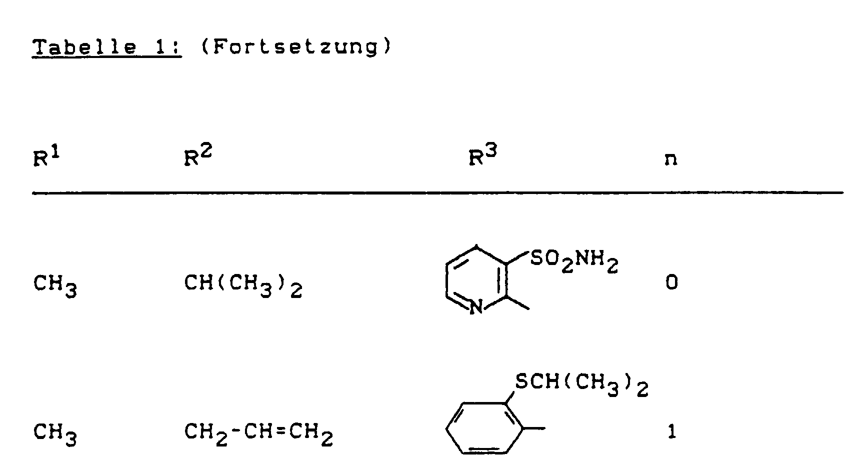

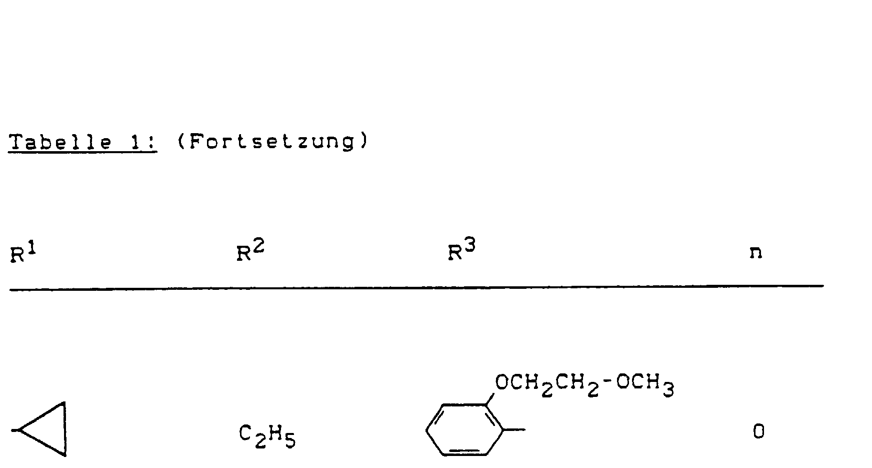

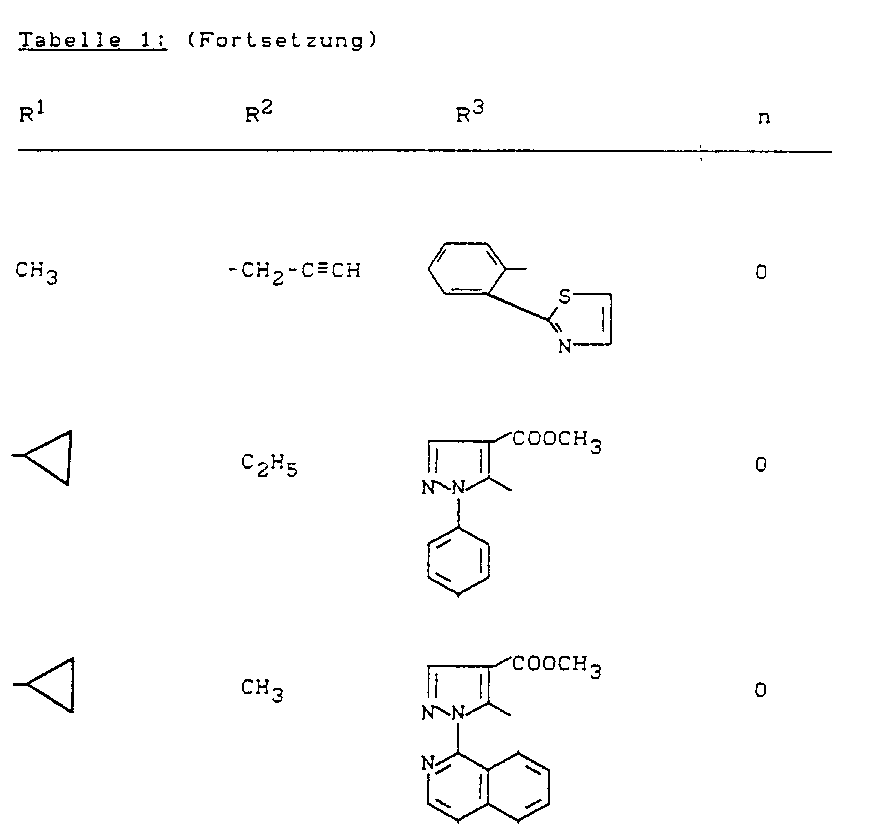

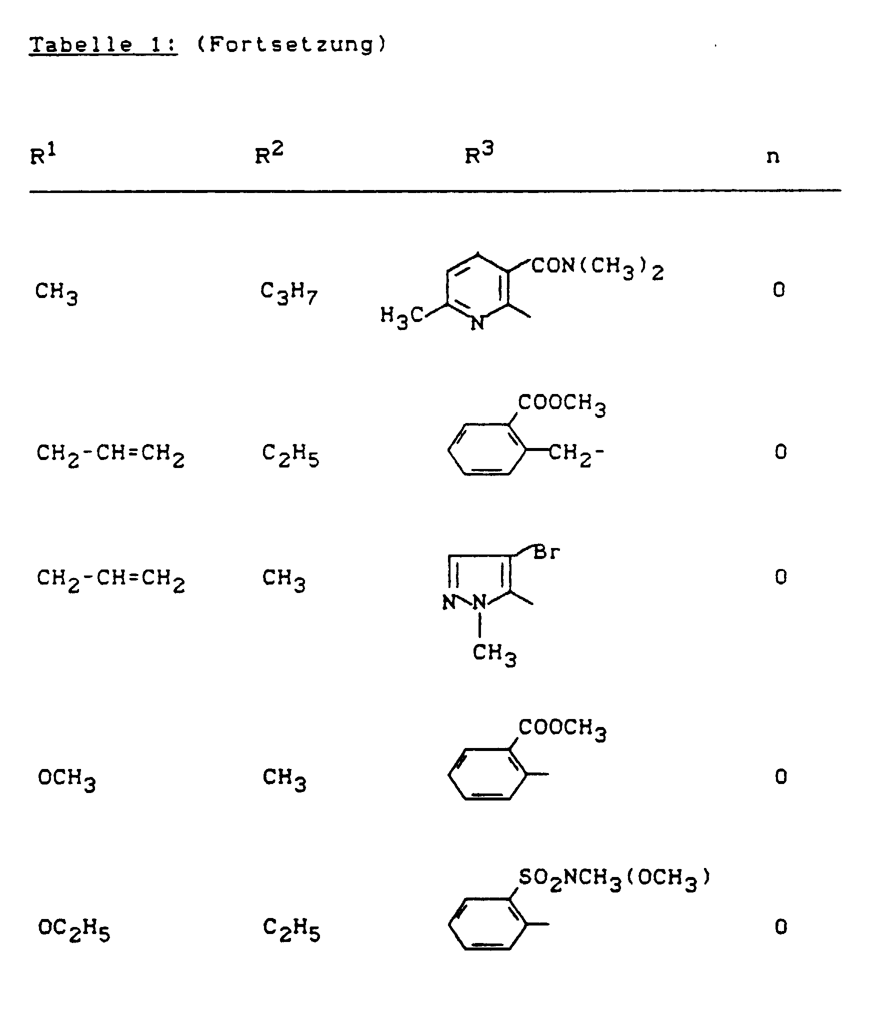

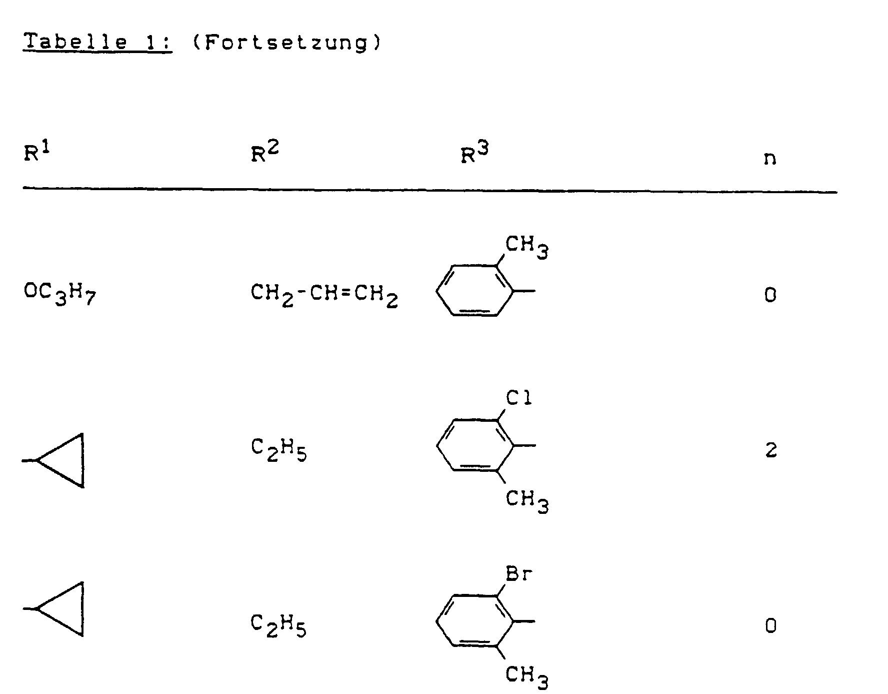

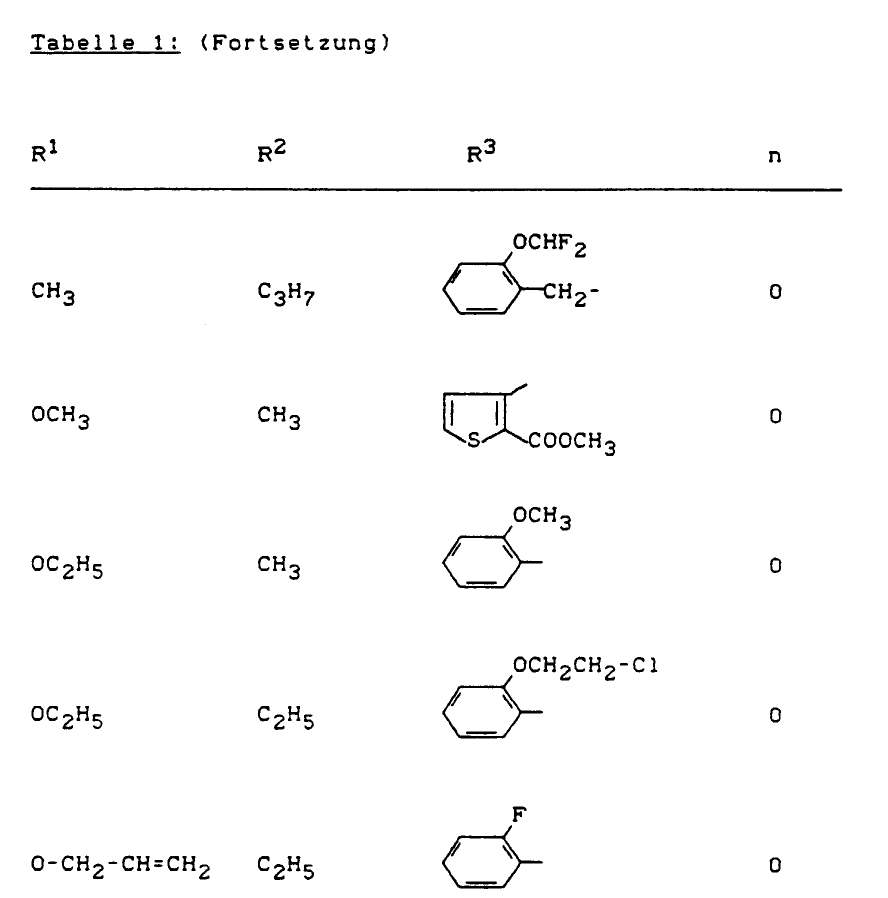

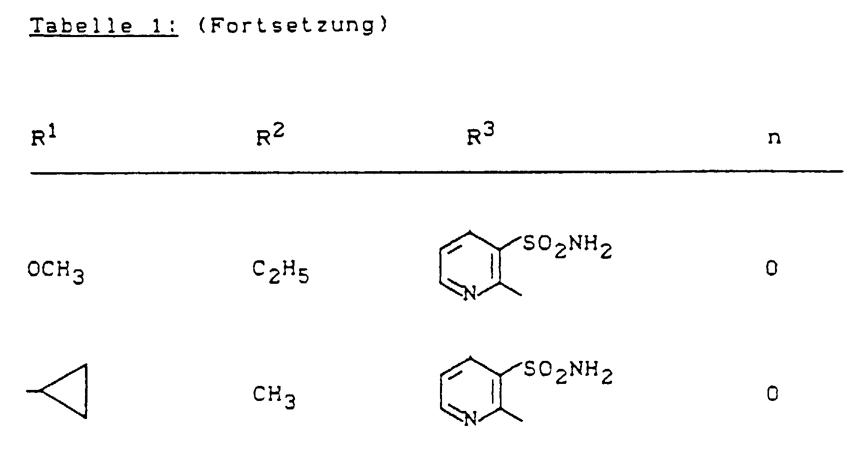

Verwendet man beispielsweise 2,6-Difluor-phenylsulfonyl isocyanat und 5-Ethylthio-4-methoxy-2,4-dihydro-3H-1,2,4-triazol-3-on als Ausgangsstoffe, so kann der Reaktionsablauf beim erfindungsgemäßen Verfahren (a) durch folgendes Formelschema skizziert werden:

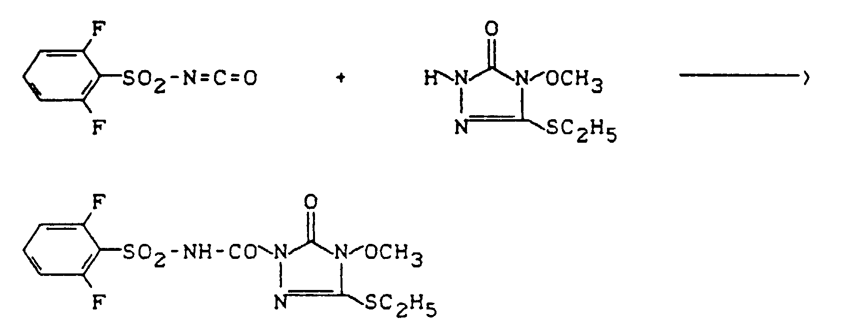

Verwendet man beispielsweise 2-Methylthio-benzolsulfonsäureamid und 2-Chlorcarbonyl-4-dimethylamino-5-propylthio-2,4-dihydro-3H-1,2,4-triazol-3-on als Ausgangsstoffe, so kann der Reaktionsablauf beim erfindungsgemäßen Verfahren (b) durch folgendes Formelschema skizziert werden (Referenzbeispiel):

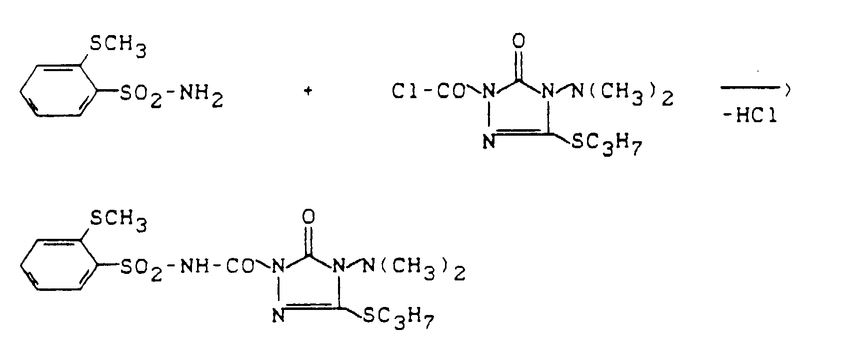

Verwendet man beispielsweise N-Methoxycarbonyl-2-methoxy-benzolsulfonsäureamid und 5-Methylsulfonyl-4-difluormethyl-2,4-dihydro-3H-1,2,4-triazol-3-on als Ausgangsstoffe, so kann der Reaktionsablauf beim erfindungsgemäßen Verfahren (c) durch das folgende Formelschema skizziert werden:

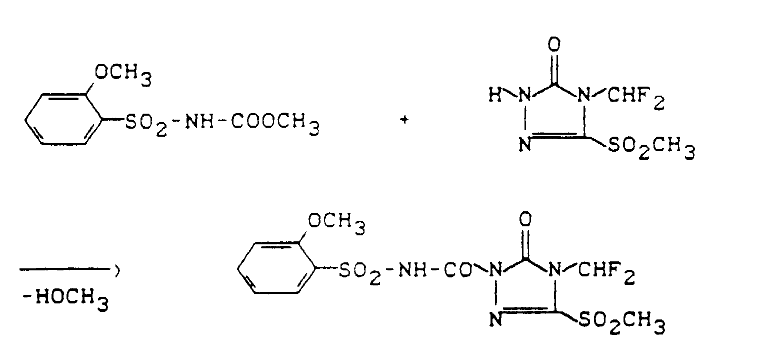

Die bei den erfindungsgemäßen Verfahren (a) und (c) zur Herstellung von Verbindungen der Formel (I) als Ausgangsstoffe zu verwendenden Triazolinone sind durch die Formel (II) allgemein definiert.

In Formel (II) haben n, R¹ und R² vorzugsweise diejenigen Bedeutungen, die bereits oben im Zusammenhang mit der Beschreibung der erfindungsgemäßen Verbindungen der Formel (I) vorzugsweise für n, R¹ und R² angegeben wurden.

Beispiele für die Ausgangsstoffe der Formel (II) sind in der nachstehenden Tabelle 2 aufgeführt.

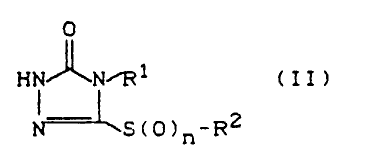

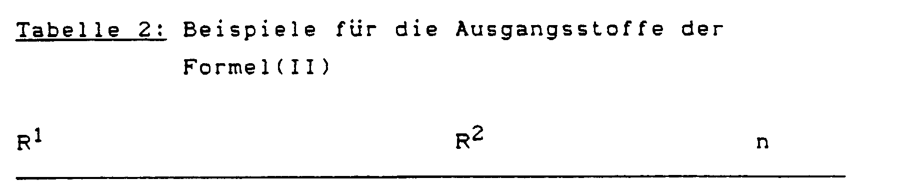

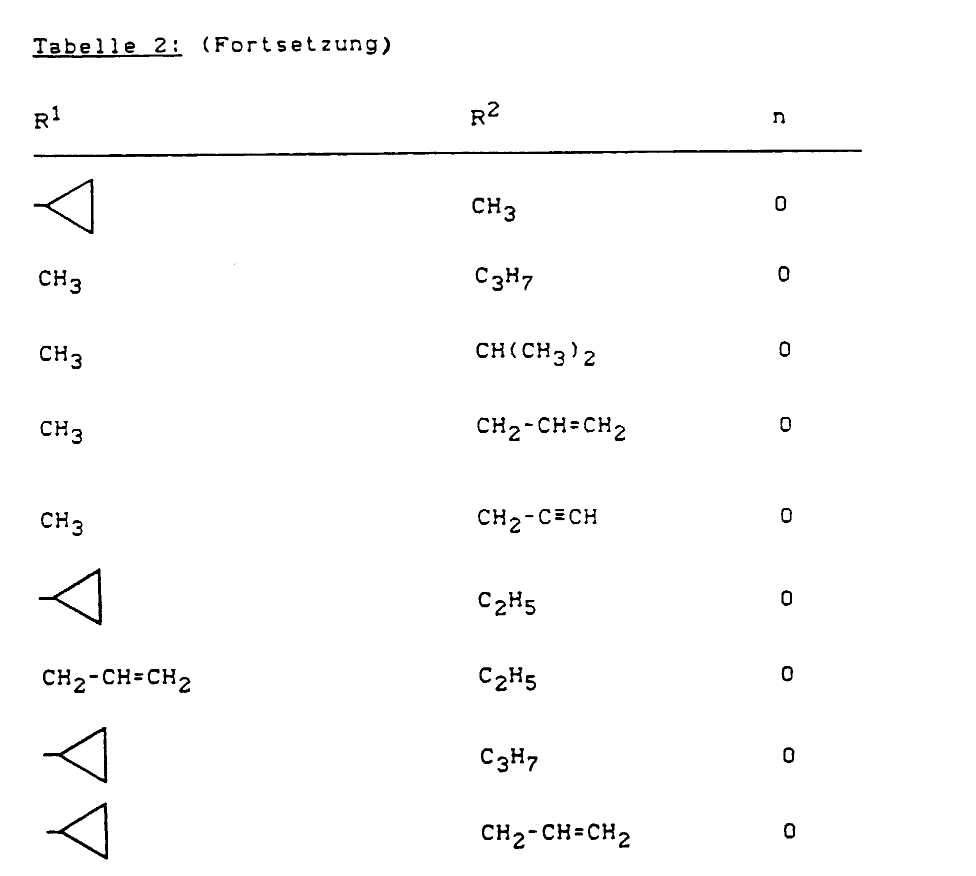

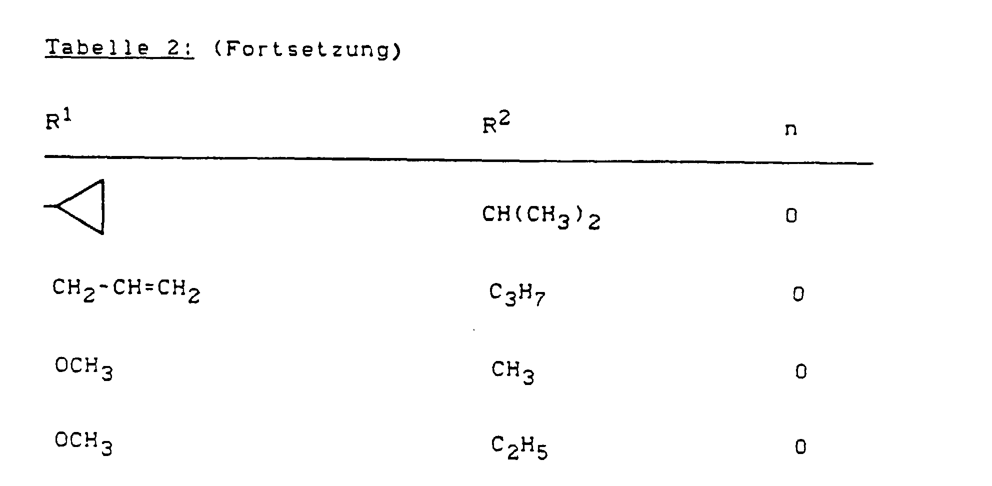

Die Ausgangsstoffe der Formel (II) sind bekannt und/oder können nach an sich bekannten Verfahren hergestellt werden (vgl. J. Heterocycl. Chem. 15 (1978), S. 377-384; DE-OS 2 250 572; DE-OS 2 527 676; DE-OS 3 709 574; US-P 4 098 896; US-P 4 110 332; JP-A-52-125 168).

Die beim erfindungsgemäßen Verfahren (a) zur Herstellung von Verbindungen der Formel (I) weiter als Ausgangsstoffe zu verwendenden Sulfonylisocyanate sind durch die Formel (III) allgemein definiert.

In Formel (III) hat R³ vorzugsweise diejenige Bedeutung, die bereits oben im Zusammenhang mit der Beschreibung der erfindungsgemäßen Verbindungen der Formel (I) vorzugsweise für R³ angegeben wurde.

Als Beispiele für die Ausgangsstoffe der Formel (III) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-phenylsulfonylisocyanat, 2-Fluor-, 2-Chlor-, 2-Difluormethoxy-, 2-Trifluormethoxy-, 2-Methoxycarbonyl- und 2-Ethoxycarbonyl-benzylsulfonylisocyanat, 2-Methoxycarbonyl-3-thienyl-sulfonylisocyanat, 4-Methoxycarbonyl- und 4-Ethoxycarbonyl-1-methyl-pyrazol-5-yl-sulfonylisocyanat.

Die Sulfonylisocyanate der Formel (III) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 80 °C.

Das erfindungsgemäße Verfahren (a) wird im allgemeinen bei Normaldruck durchgeführt.

Zur Durchführung des erfindungsgemäßen Verfahrens (a) setzt man je Mol Triazolinon der Formel (II) im allgemeinen zwischen 1 und 3 Mol, vorzugsweise zwischen 1 und 2 Mol, Sulfonylisocyanat der Formel (III) ein.

Die Reaktionskomponenten können in beliebiger Reihenfolge zusammengegeben werden. Das Reaktionsgemisch wird bis zum Ende der Umsetzung gerührt und das Produkt durch Absaugen isoliert. In einer anderen Aufarbeitungsvariante wird eingeengt und das im Rückstand verbleibende Rohprodukt mit einem geeigneten Lösungsmittel, wie z. B. Diethylether, zur Kristallisation gebracht. Das hierbei kristallin angefallene Produkt der Formel (I) wird durch Absaugen isoliert.

Die beim erfindungsgemäßen Verfahren (b) zur Herstellung von Verbindungen der Formel (I) als Ausgangsstoffe zu verwendenden Triazolinon-Derivate sind durch die Formel (IV) allgemein definiert.

In Formel (IV) haben n, R¹ und R² vorzugsweise diejenigen Bedeutungen, die bereits oben im Zusammenhang mit der Beschreibung der erfindungsgemäßen Verbindungen der Formel (I) vorzugsweise für n, R¹ und R² angegeben wurden und
- Z: steht vorzugsweise für Methoxy oder Phenoxy.

Mögliche Beispiele für die Ausgangsstoffe der Formel (IV) sind die aus den in Tabelle 2 aufgeführten Verbindungen der Formel (II) und Phosgen, Chlorameisensäuremethylester, Chlorameisensäure-benzylester, Chlorameisensäurephenylester oder Diphenylcarbonat herzustellenden Verbindungen der Formel (IV).

Die Ausgangsstoffe der Formel (IV) sind noch nicht bekannt.

Man erhält die neuen Triazolinon-Derivate der Formel (IV), wenn man Triazolinone der allgemeinen Formel (II) 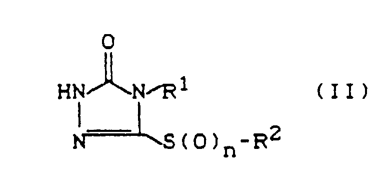 in welcher
- n, R¹ und R²: die oben angegebenen Bedeutungen haben, mit Kohlensäurederivaten der allgemeinen Formel (XI)

Z-CO-Z¹ (XI)

in welcher
- Z: die oben angegebene Bedeutung hat und
- Z¹: für eine Abgangsgruppe wie Chlor, Methoxy, Benzyloxy oder Phenoxy steht,
gegebenenfalls in Gegenwart eines Verdünnungsmittels, wie z.B. Tetrahydrofuran, und gegebenenfalls in Gegenwart eines Säureakzeptors, wie z.B. Natriumhydrid oder Kalium-tert-butylat, bei Temperaturen zwischen -20°C und +100°C umsetzt (vgl, auch die Herstellungsbeispiele).

Die beim erfindungsgemäßen Verfahren (b) zur Herstellung von Verbindungen der Formel (I) weiter als Ausgangsstoffe zu verwendenden Sulfonsäureamide sind durch die Formel (V) allgemein definiert.

In Formel (V) hat R³ vorzugsweise diejenige Bedeutung, die bereits oben im Zusammenhang mit der Beschreibung der erfindungsgemäßen Verbindungen der Formel (I) vorzugsweise für R³ angegeben wurde.

Als Beispiele für die Ausgangsstoffe der Formel (V) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-benzolsulfonsäureamid, 2-Fluor-, 2-Chlor-, 2-Difluormethoxy-, 2-Trifluormethoxy-, 2-Methoxycarbonyl- und 2-Ethoxycarbonyl-phenylmethansulfonsäureamid, 2-Methoxycarbonyl-3-thiophensulfonsäureamid, 4-Methoxycarbonyl-und 4-Ethoxycarbonyl-1-methyl-pyrazol-5-sulfonsäureamid.

Die Sulfonsäureamide der Formel (V) sind bekannt und/ oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Betracht, wie sie beispielsweise oben für das erfindungsgemäße Verfahren (a) angegeben sind.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) zur Herstellung von Verbindungen der Formel (I) als Ausgangsstoffe zu verwendenden Triazolinone der Formel (II) sind bereits als Ausgangsstoffe für das erfindungsgemäße Verfahren (a) beschrieben worden.

Die beim erfindungsgemäßen Verfahren (c) zur Herstellung von Verbindungen der Formel (I) weiter als Ausgangsstoffe zu verwendenden Sulfonsäureamid-Derivate sind durch die Formel (VI) allgemein definiert.

In Formel (VI) haben R³ und Z vorzugsweise diejenigen Bedeutungen, die bereits oben im Zusammenhang mit der Beschreibung der erfindungsgemäßen Verbindungen der Formel (I) bzw. (IV) vorzugsweise für R³ und Z angegeben wurden.

Das erfindungsgemäße Verfahren (c) wird vorzugsweise unter Verwendung von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Verfahren (c) wird gegebenenfalls in Gegenwart eines Säureakzeptors durchgeführt. Es kommen hierbei die gleichen Säurebindemittel in Betracht, die oben im Zusammenhang mit der Beschreibung des erfindungsgemäßen Verfahrens (b) genannt wurden.

Die Reaktionstemperaturen können bei dem erfindungsgemäßen Verfahren (c) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Zur Überführung in Salze werden die Verbindungen der Formel (I) mit geeigneten Salzbildnern, wie z.B. Natrium- oder Kalium-hydroxid, -methylat oder -ethylat, Ammoniak, Isopropylamin, Dibutylamin oder Triethylamin, in geeigneten Verdünnungsmitteln, wie z.B. Wasser, Methanol oder Ethanol, verrührt. Die Salze können dann - gegebenenfalls nach Einengen - als kristalline Produkte isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren. Sie sind deutlich wirksamer als z.B. Isocarbamid.

In gewissem Umfang zeigen die erfindungsgemäßen Verbindungen auch fungizide Wirkung, z.B. gegen Pyricularia an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridin-yl)oxy]-essigsäure bzw, deren 1-Methylheptylester (FLUROXYPYR); N-Phosphonomethylglycin (GLYPHOSATE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbamat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]thiophen-2-carbonsäure-methylester (THIAMETURON). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen her vor.

### Herstellungsbeispiele

### Referenzbeispiel 1

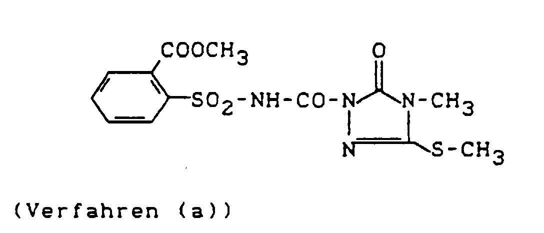

3,0 g (20,7 mMol) 4-Methyl-5-methylthio-2,4-dihydro-3H-1,2,4-triazol-3-on werden in 60 ml Acetonitril gelöst und unter Rühren werden 7,0 g (29 mMol) 2-Methoxycarbonyl-phenylsulfonylisocyanat, gelöst in 20 ml Acetonitril, zu dieser Lösung gegeben. Das Reaktionsgemisch wird 6 Stunden bei 20°C gerührt. Dann wird das kristallin angefallene Produkt durch Absaugen isoliert.

Man erhält 6,0 g (75 % der Theorie) 4-Methyl-5-methylthio-2-(2-methoxycarbonyl-phenylsulfonyl-aminocarbonyl)-2,4-dihydro-3H-1,2,4-triazol-3-on vom Schmelzpunkt 184°C.

### Referenzbeispiel 2

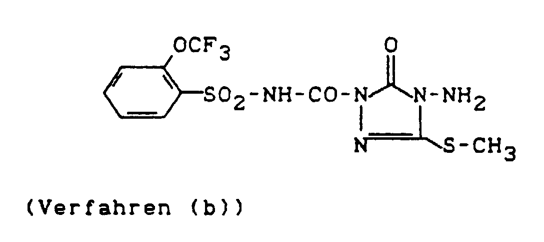

1,7 g (11,2 mMol) 1,8-Diazabicyclo-[5,4,0]-undec-7-en (DBU) und 2,7 g (11,2 mMol) 2-Trifluormethoxybenzol-sulfonamid werden zu einer Lösung von 3,0 g (11,3 mMol) 4-Amino-5-methylthio-2-phenoxy-carbonyl-2,4-dihydro-3H-1,2,4-triazol-3-on in 60 ml Methylenchlorid gegeben. Das Reaktionsgemisch wird 4 Stunden bei 20°C gerührt, dann zweimal mit 1 %iger Salzsäure und dreimal mit Wasser gewaschen, mit Natriumsulfat getrocknet und filtriert. Das Filtrat wird eingeengt, der Rückstand mit Diethylether verrührt und das kristallin angefallene Produkt durch Absaugen isoliert.

Man erhält 2,1 g (45 % der Theorie) 4-Amino-5-methylthio-2-(2-trifluormethoxy-phenylsulfonyl-aminocarbonyl)-2,4-dihydro-3H-1,2,4-triazol-3-on vom Schmelzpunkt 136°C.

Analog zu den Referenzbeispielen 1 und 2 sowie entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

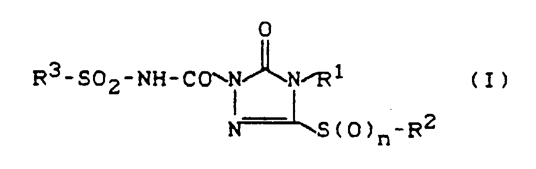

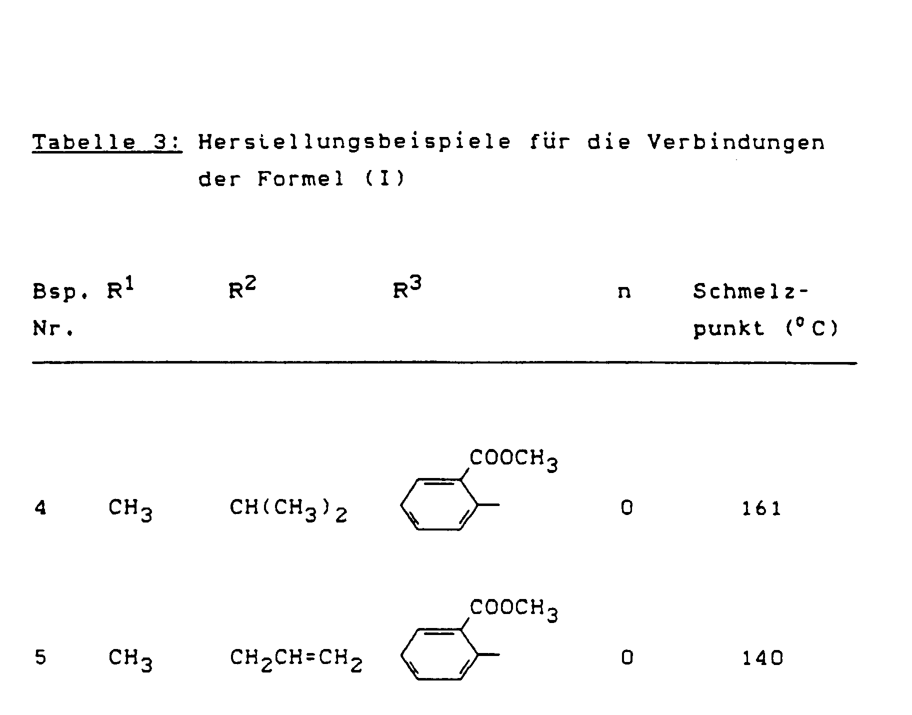

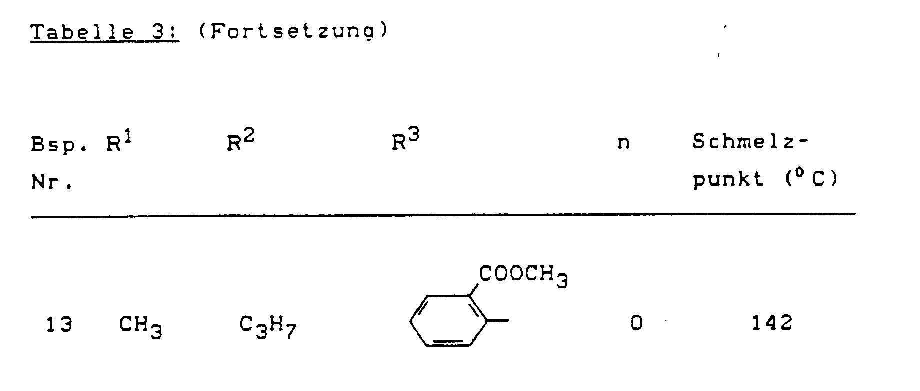

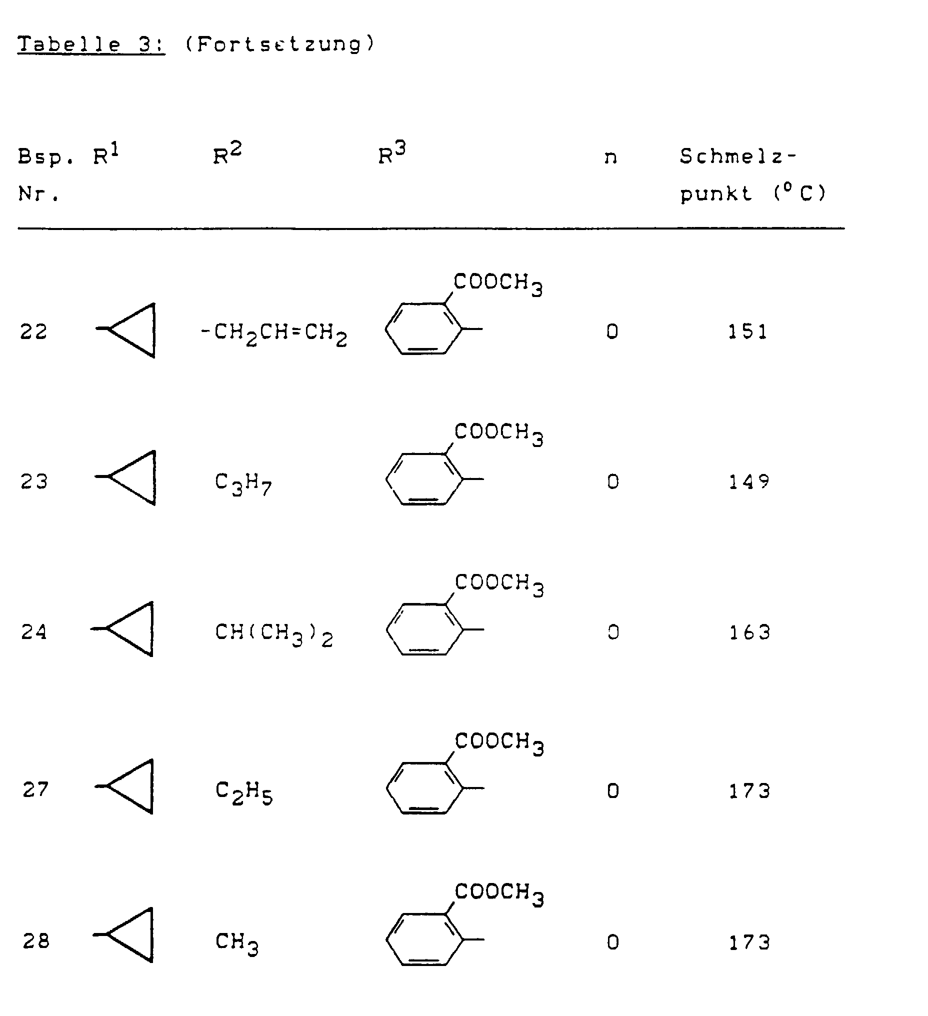

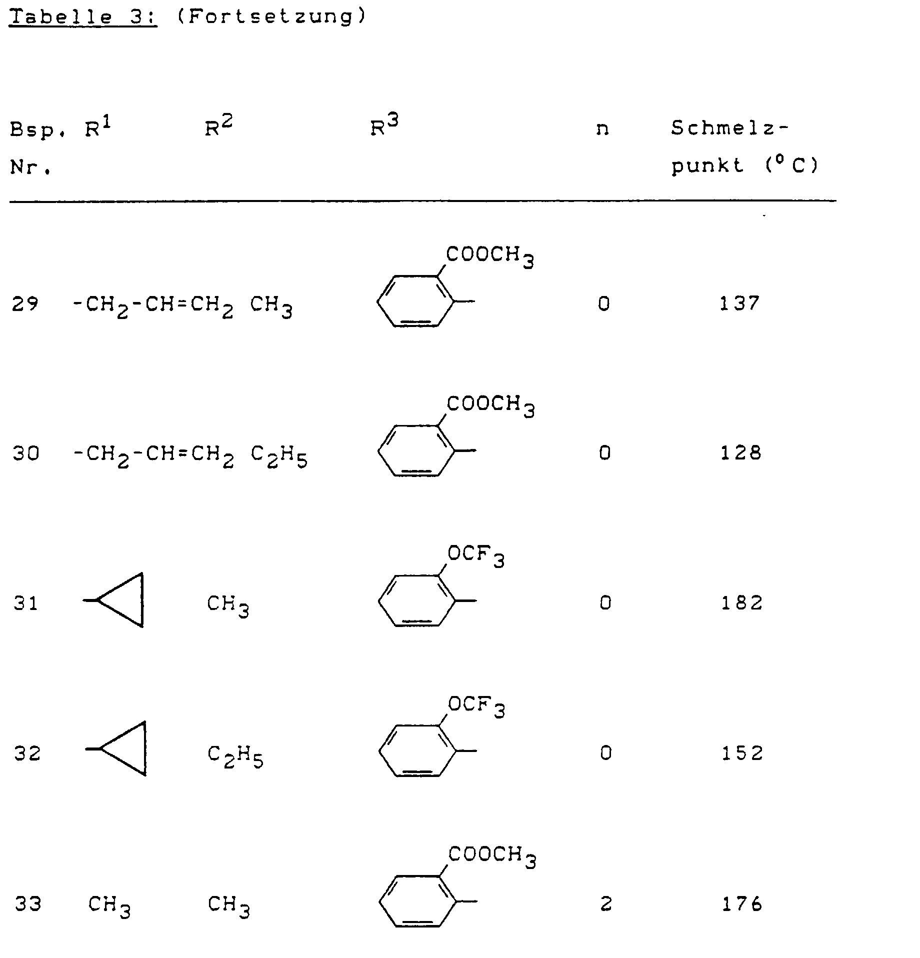

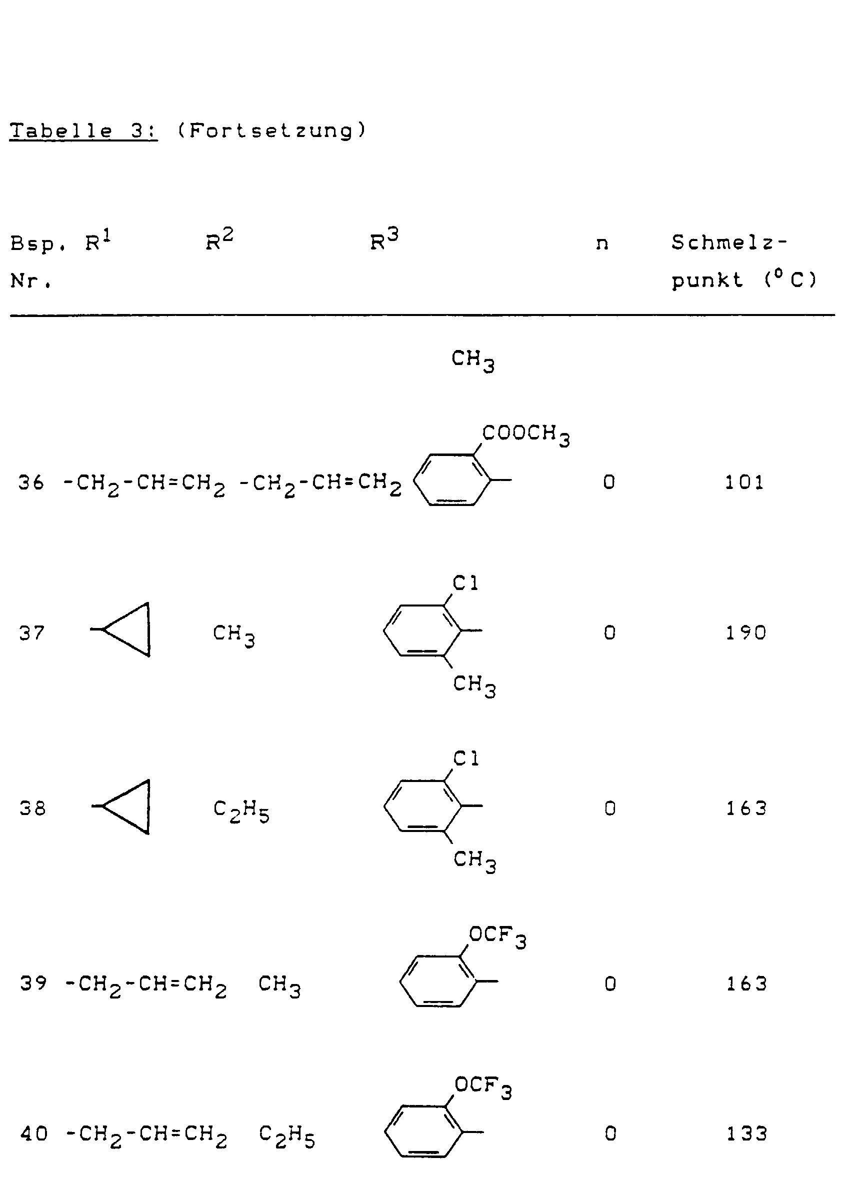

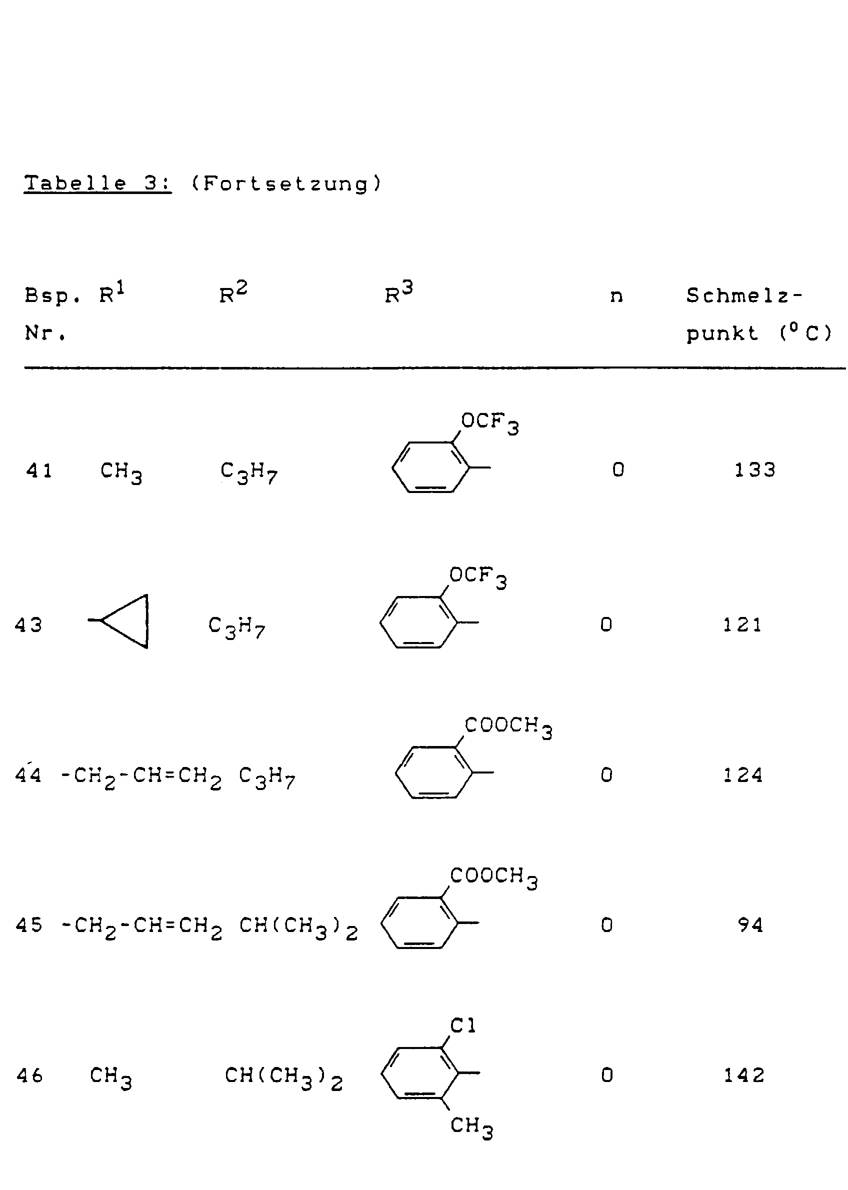

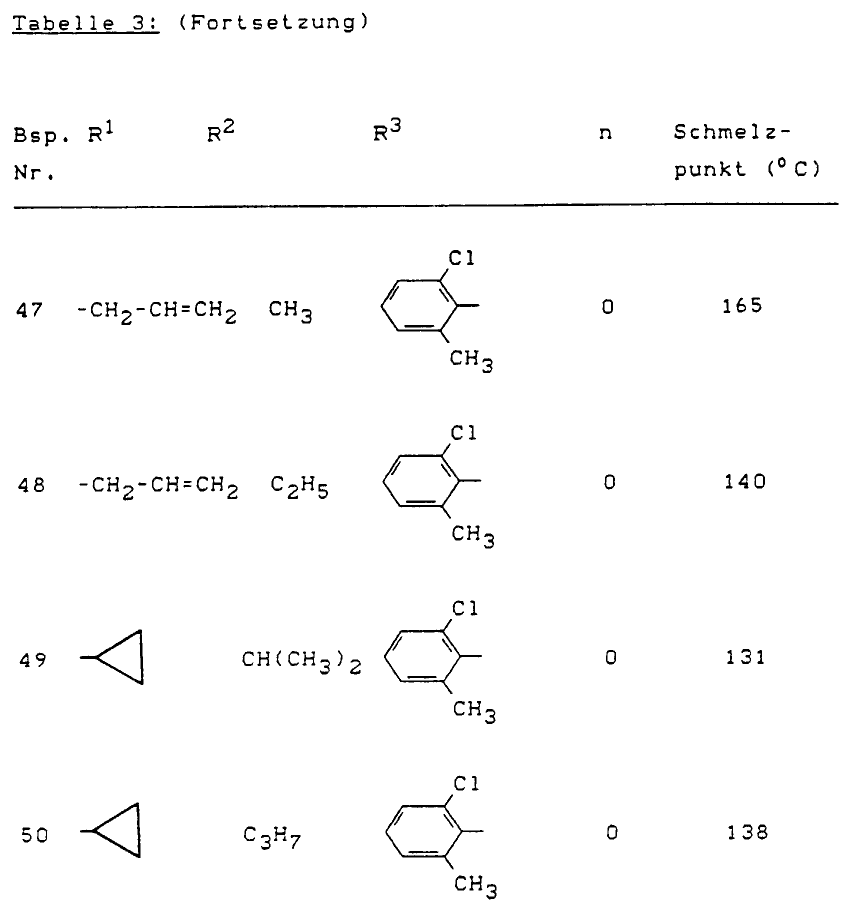

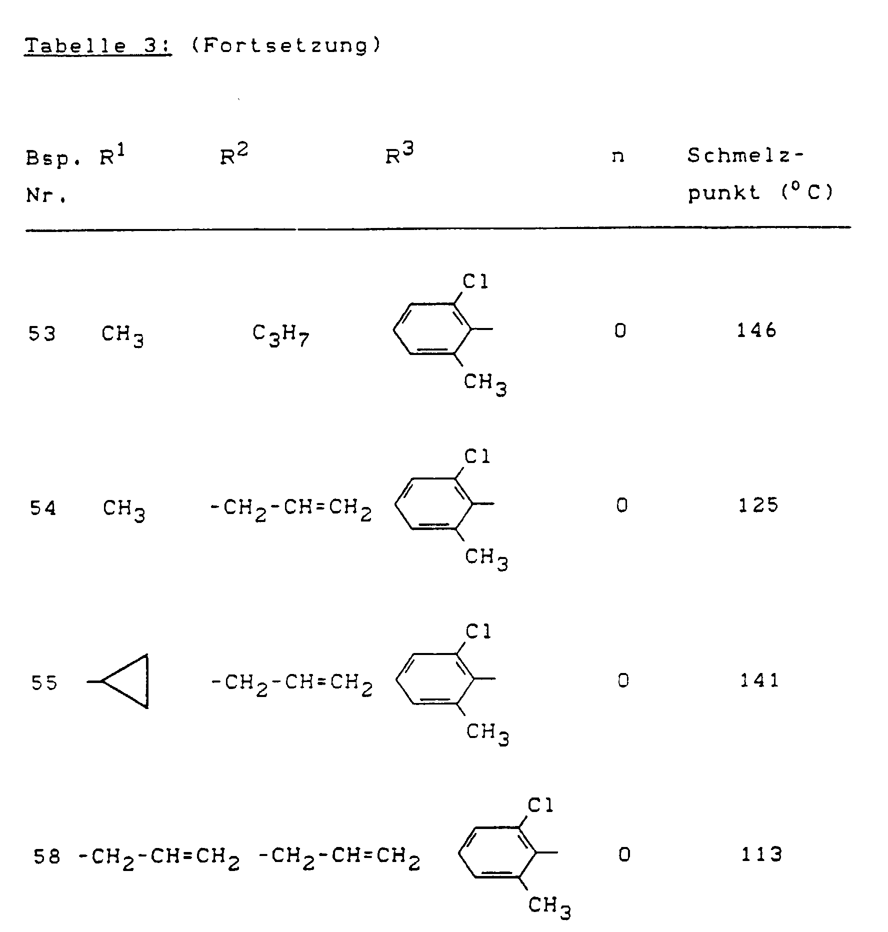

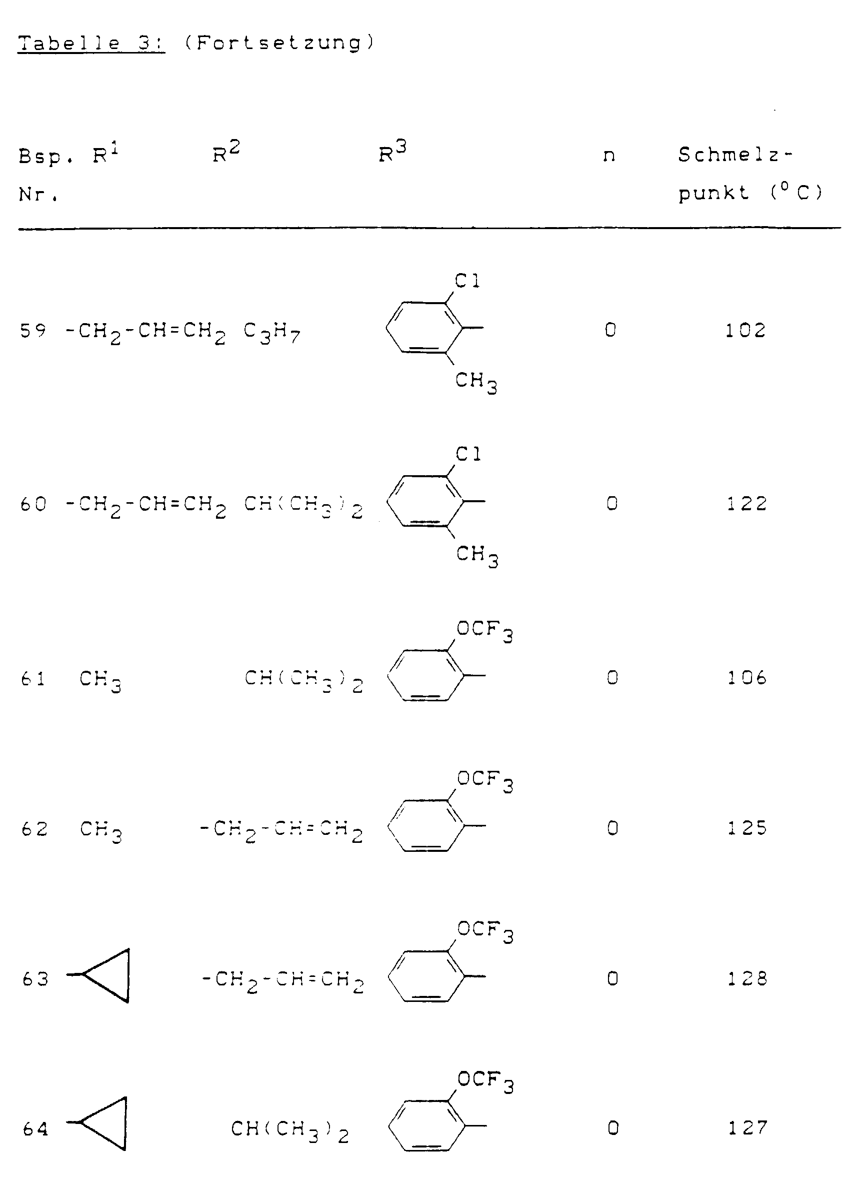

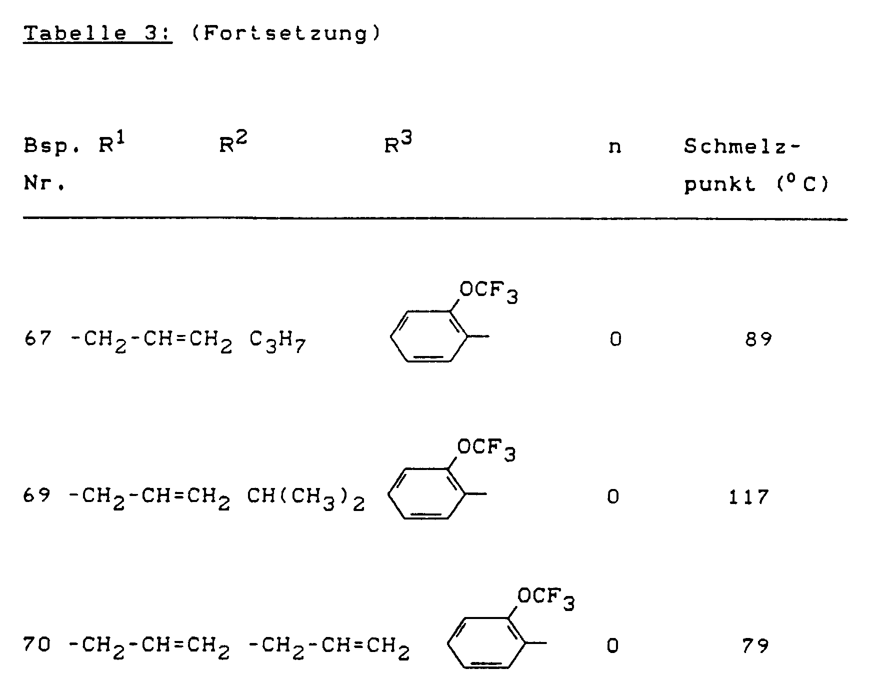

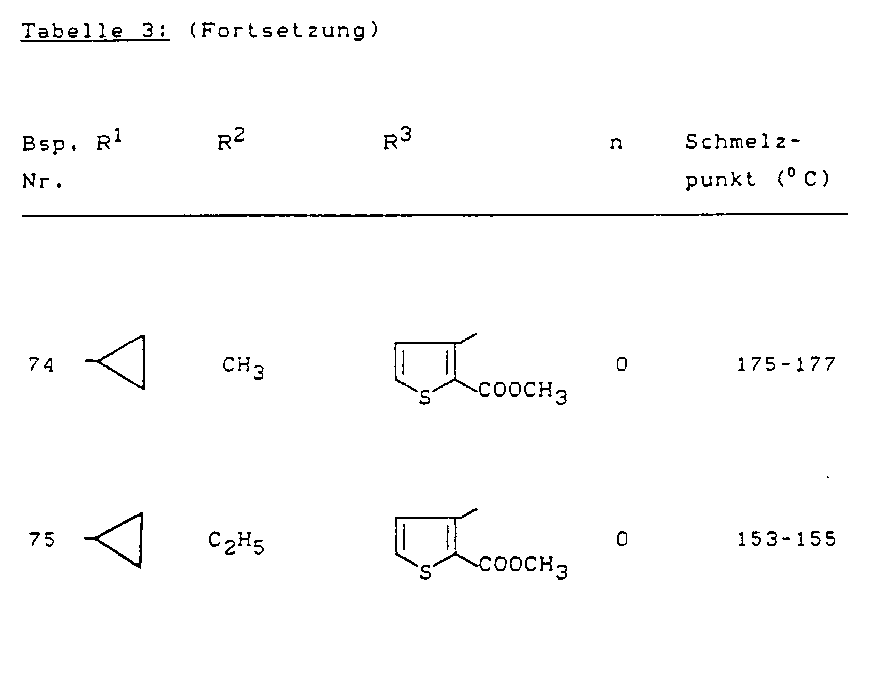

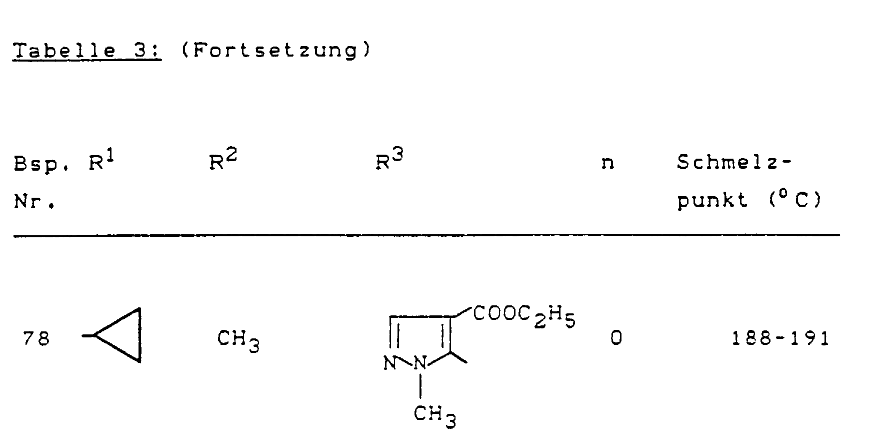

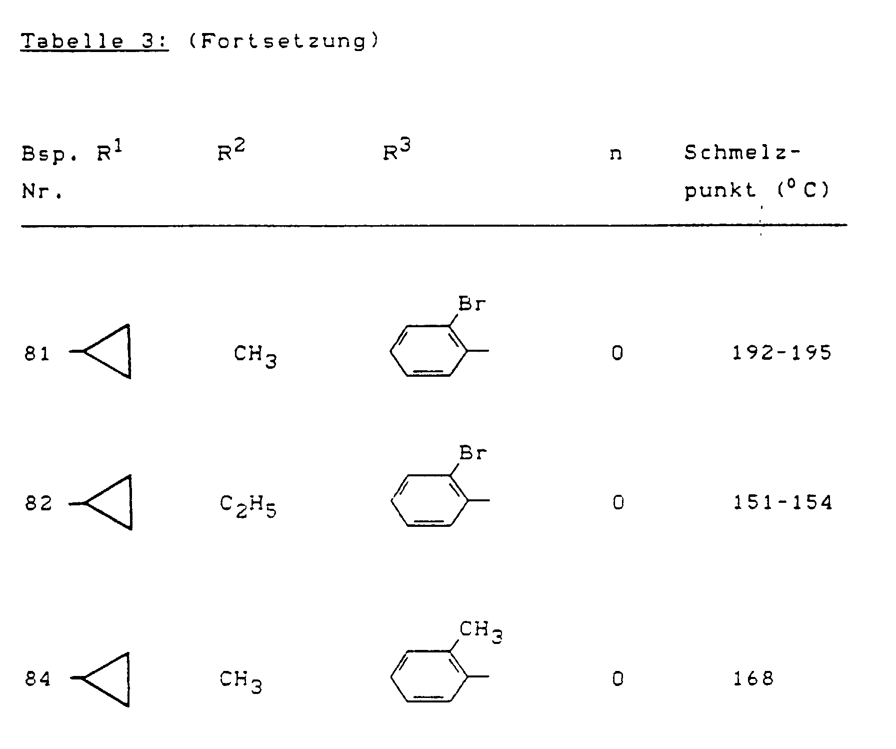

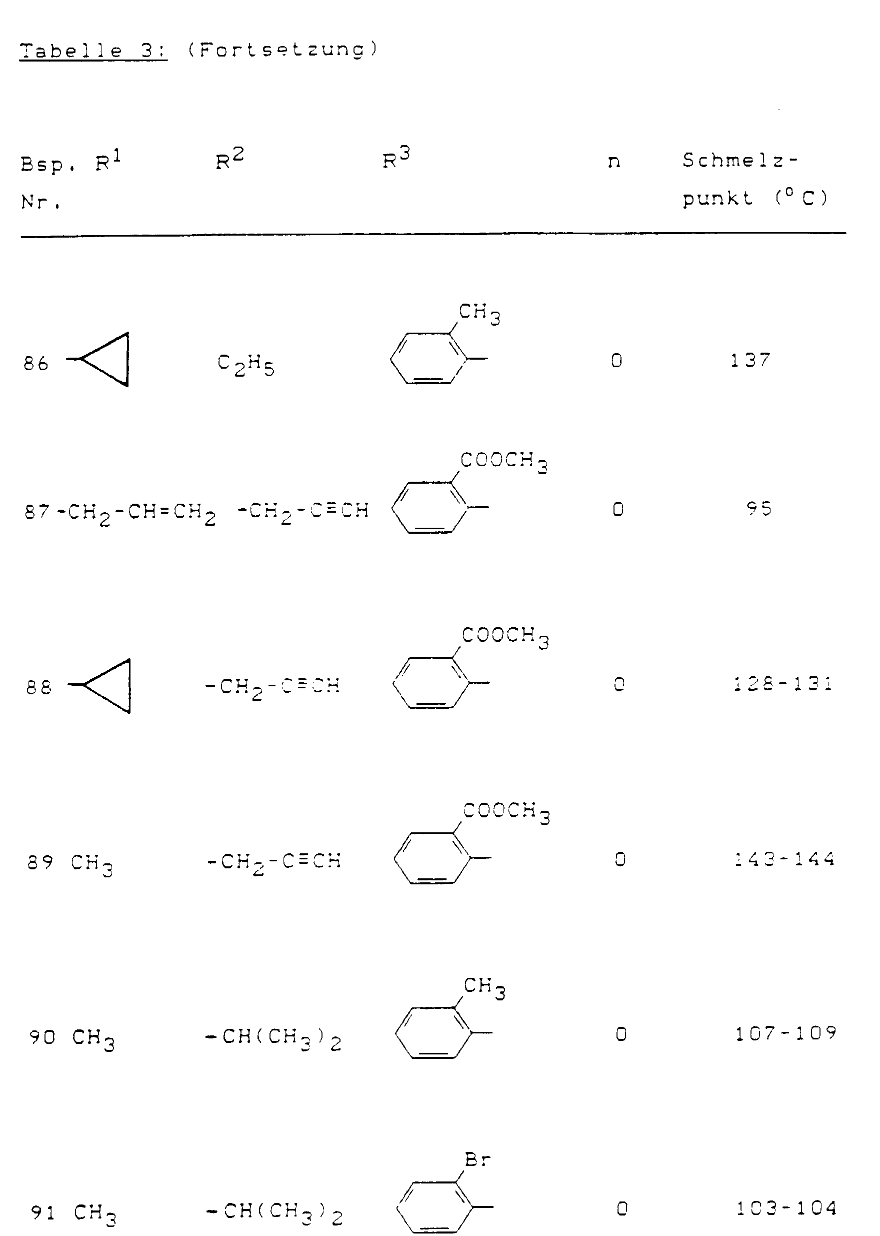

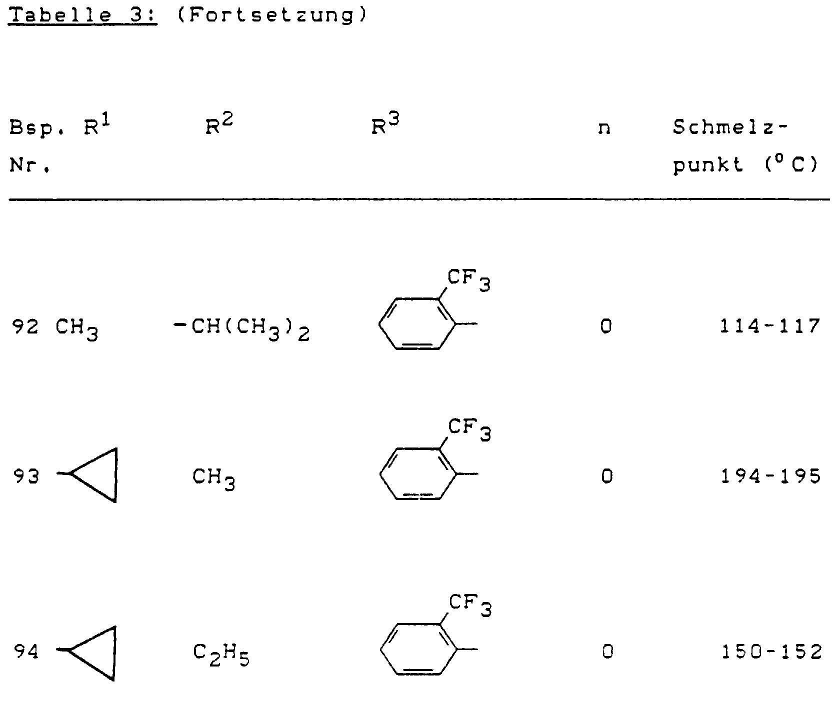

### Ausgangsstoffe der Formel (II)

### Referenzbeispiel (II-1)

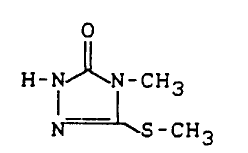

### 1. Stufe

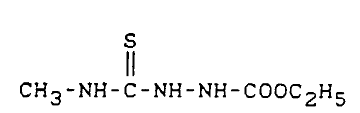

Eine Lösung von 175 g (2,4 Mol) Methylisothiocyanat in 300 ml Diethylether wird unter Rühren zu einer Lösung von 250 g (2,4 Mol) Hydrazinoameisensäureethylester gegeben. Das Reaktionsgemisch wird 12 Stunden bei 20°C gerührt, dann auf ca. 10°C abgekühlt und das kristallin angefallene Produkt durch Absaugen isoliert. Man erhält 404 g (95 % der Theorie) 4-Methyl-1-ethoxycarbonylthiosemicarbazid vom Schmelzpunkt 130°C.

Analog erhält man:
4-Cyclopropyl-1-methoxycarbonyl-thiosemicarbazid (Schmelzpunkt: 148°C);
4-Allyl-1-methoxycarbonyl-thiosemicarbazid (Schmelzpunkt: 151° C).

### Stufe 2

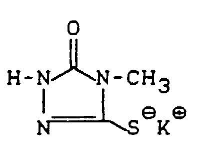

Eine Mischung aus 10,0 g (56,5 mMol) 4-Methyl-1-ethoxycarbonyl-thiosemicarbazid (vgl. 1. Stufe), 4,0 g (29 mMol) Kaliumcarbonat und 80 ml Ethanol wird bis zur Beendigung der Gasentwicklung unter Rückfluß zum Sieden erhitzt (ca. 3 Stunden). Nach dem Erkalten wird eingeengt, der Rückstand mit 50 ml Methylenchlorid verrührt und das ungelöst gebliebene Produkt durch Absaugen isoliert.

Man erhält 9,2 g (96 % der Theorie) des Kaliumsalzes von 5-Mercapto-4-methyl-2,4-dihydro-3H-1,2,4-triazol-3-on (Schmelztemperatur >230°C).

Analog erhält man die Kaliumsalze von 5-Mercapto-4-cyclopropyl-2,4-dihydro-3H-1,2,4-triazol-3-on und 5-Mercapto-4-allyl-2,4-dihydro-3H-1,2,4-triazol-3-on, welche alle oberhalb von 230°C schmelzen.

### 3. Stufe

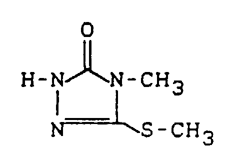

Eine Mischung aus 4,0 g (23,7 mMol) 5-Mercapto-4-methyl-2,4-dihydro-3H-1,2,4-triazol-3-on-Kaliumsalz (vgl. 2. Stufe), 4,1 g (28,9 mMol) Methyliodid und 80 ml Methanol wird 12 Stunden bei 20°C gerührt. Dann wird eingeengt, der Rückstand mit Methylenchlorid verrührt und das ungelöst gebliebene Kaliumiodid durch Filtration abgetrennt. Das Filtrat wird eingeengt, der Rückstand mit 500 ml Diethylether/Petrolether (Vol. 1:1) verrührt und das kristallin angefallene Produkt durch Absaugen isoliert.

Man erhält 3,4 g (99 % der Theorie) 4-Methyl-5-methylthio-2,4-dihydro-3H-1,2,4-triazol-3-on vom Schmelzpunkt 97°C.

Analog Referenzbeispiel (II-1) können beispielsweise auch die in der nachstehenden Tabelle 4 aufgeführten Verbindungen der Formel (II) hergestellt werden.

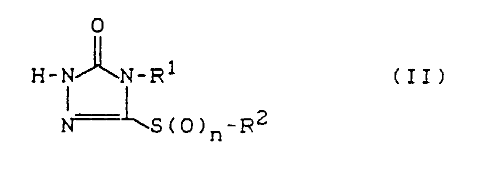

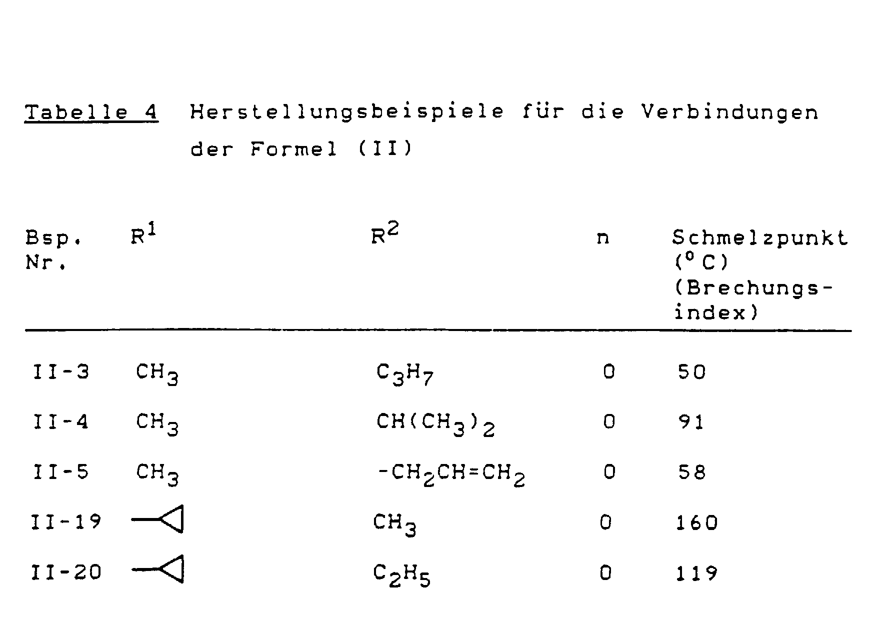

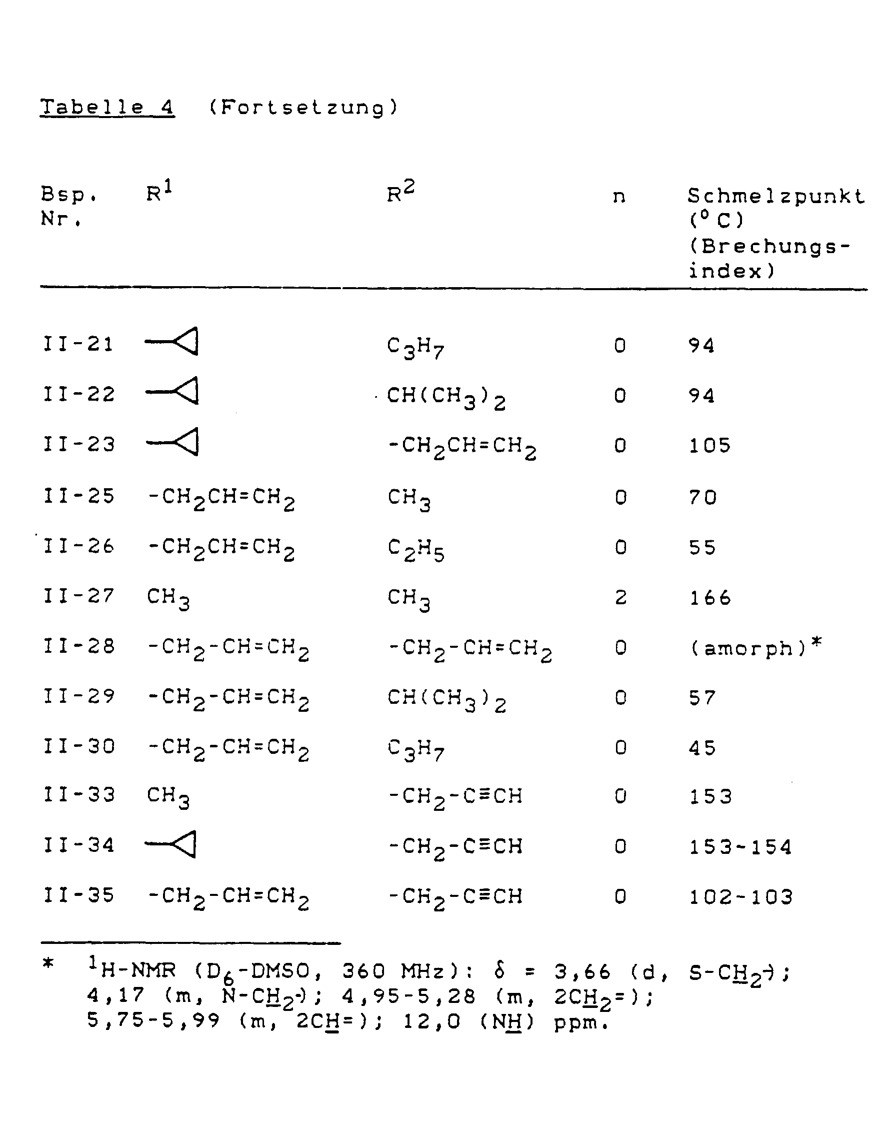

### Ausgangsstoffe der Formel (IV):

### Referenzbeispiel (IV-1)

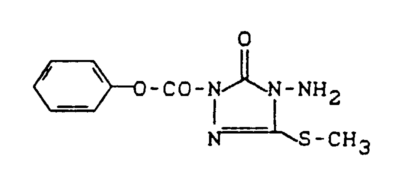

Eine Mischung aus 20,0 g (0,137 Mol) 4-Amino-5-methylthio-2,4-dihydro-3H-1,2,4-triazol-3-on, 5,6 g (0,141 Mol) Natriumhydroxid, 0,2 g Tetrabutylammoniumbromid und 200 ml Methylenchlorid/Wasser (Vol. 1:1) wird unter starkem Rühren mit 22,1 g (0,141 Mol) Chlorameisensäurephenylester versetzt, Das Reaktionsgemisch wird 12 Stunden bei 20°C gerührt und das kristallin angefallene Produkt durch Absaugen isoliert. Man erhält 34,8 g (95 % der Theorie) 4-Amino-5-methylthio-2-phenoxycarbonyl-2,4-dihydro-3H-1,2,4-triazol-3-on vom Schmelzpunkt 211°C.

Analog Referenzbeispiel (IV-1) können beispielsweise auch die in der nachstehenden Tabelle 5 aufgeführten Verbindungen der Formel (IV) hergestellt werden.

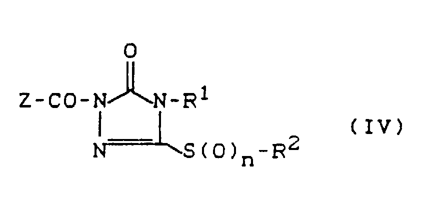

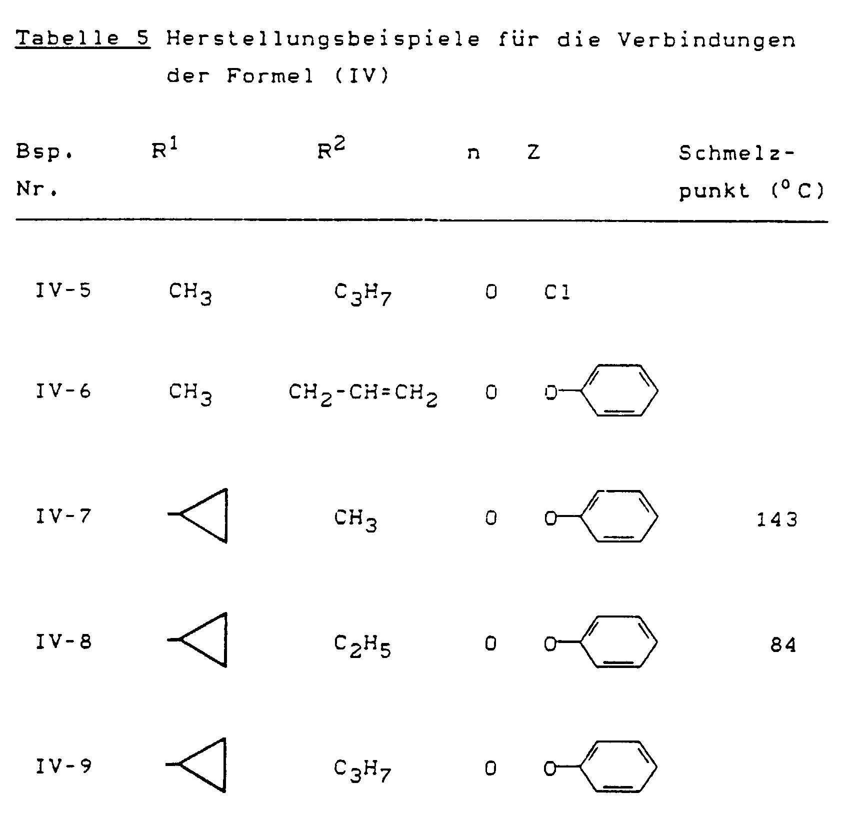

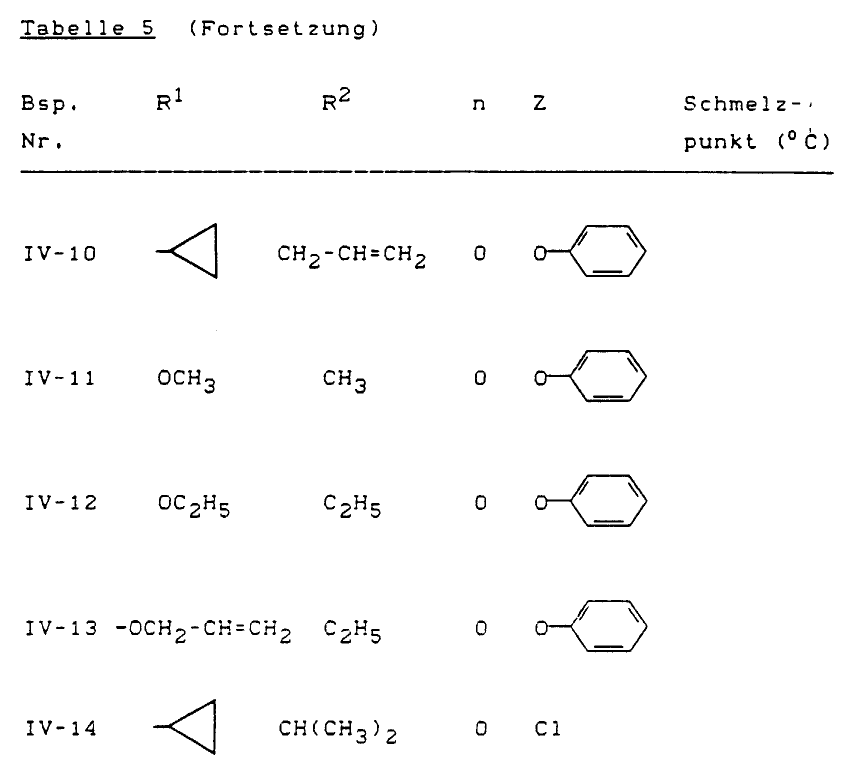

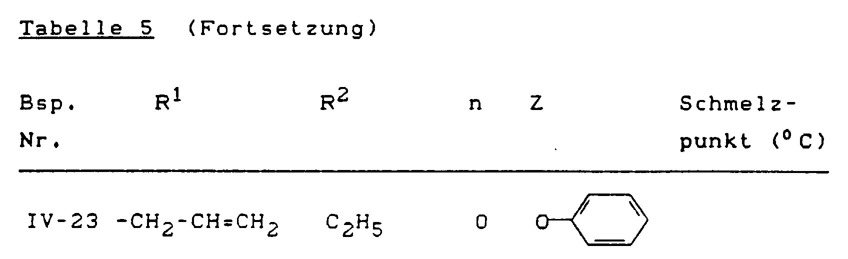

### Anwendungsbeispiele:

Bei den folgenden Anwendungsbeispielen wird das bekannte Herbizid Isocarbamid nachstehender Formel (A) als Vergleichssubstanz herangezogen: 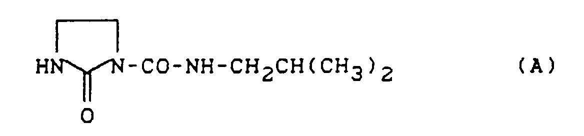 (bekannt z.B. aus DE-17 95 117).

Die Formeln der für die Anwendungsbeispiele herangezogenen erfindungsgemäßen Verbindungen sind - mit der Numerierung der Herstellungsbeispiele - nachstehend einzeln aufgeführt:

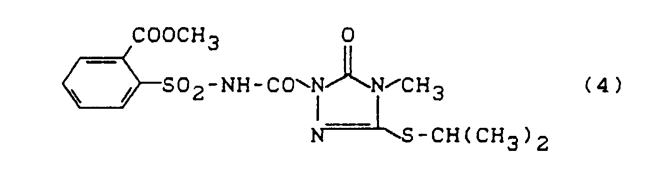

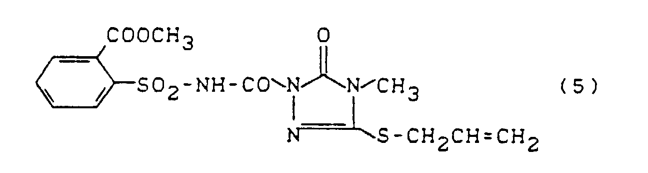

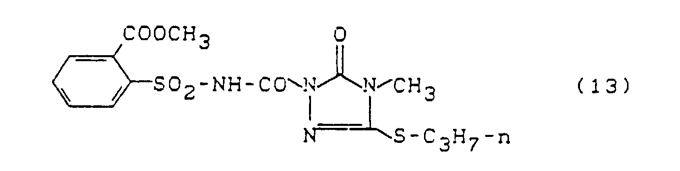

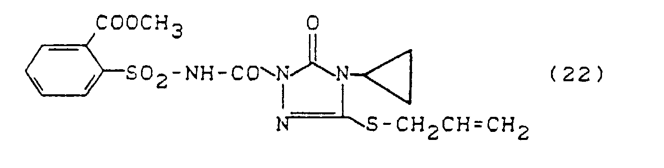

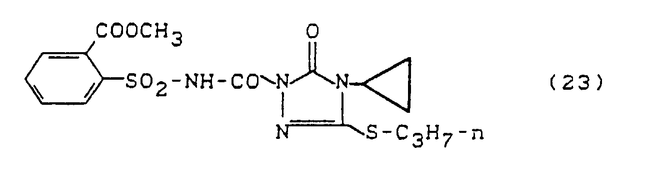

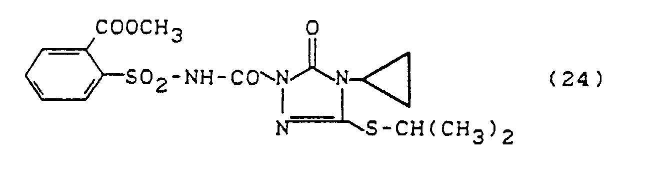

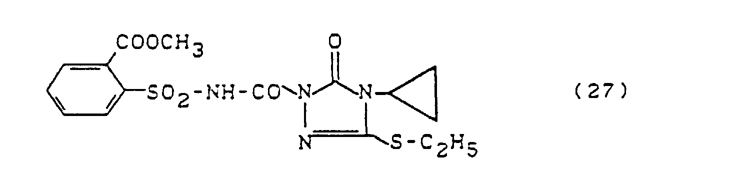

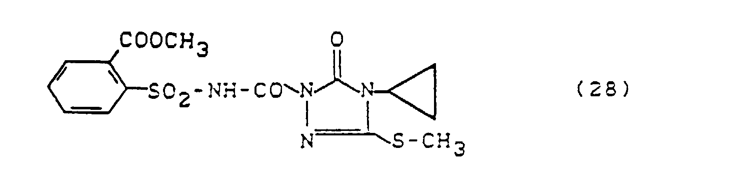

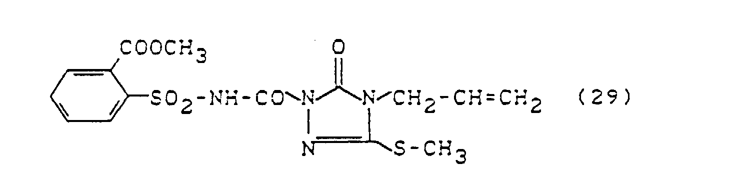

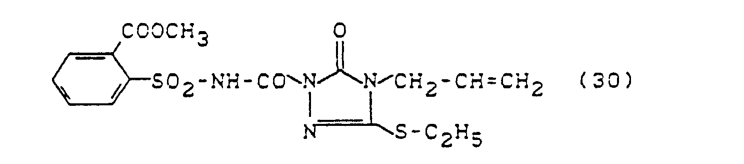

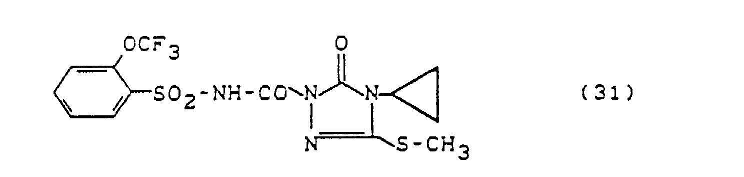

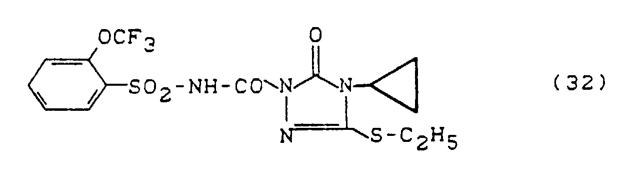

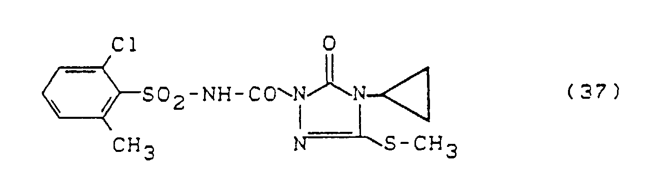

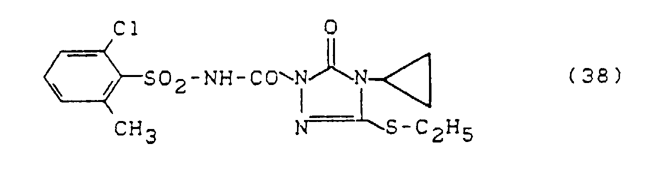

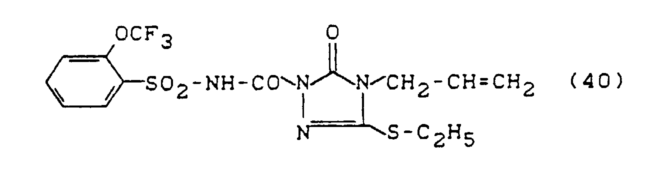

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration,

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle, Es bedeuten:
- 0 % =: keine Wirkung (wie unbehandelte Kontrolle)
- 100 % =: totale Vernichtung

Eine deutliche Überlegenheit in der Wirksamkeit gegenüber dem Stand der Technik zeigen in diesem Test z.B. die Verbindungen gemäß den Herstellungsbeispielen 4, 5, 13, 22, 23, 24, 27, 28, 29, 30, 31, 32, 37.

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

Eine deutliche Überlegenheit in der Wirksamkeit gegenüber dem Stand der Technik zeigen in diesem Test z.B. die Verbindungen gemäß den Herstellungsbeispielen 4, 5, 13, 22, 23, 24, 27, 28, 29, 30, 31, 32, 37, 38, 40.

### Beispiel C

### Pyricularia-Test (Reis) /protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefall.

Eine gute Wirkung zeigen in diesem Test z.B. die Verbindungen gemäß folgender Herstellungsbeispiele: 4, 5.

### Beispiel D

### Pyricularia-Test (Reis)/systemisch

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegeben Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine ausgezeichnete Wirksamkeit zeigen in diesem Test z.B. die Verbindungen gemäß folgender Herstellungsbeispiele: 4, 5, 22.

## Patentansprüche

1. Sulfonylaminocarbonyltriazolinone mit über Schwefel gebundenen Substituenten der allgemeinen Formel (I), 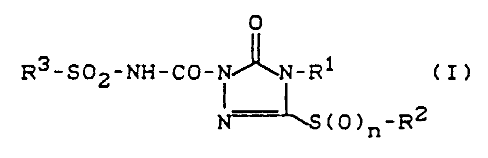 in welcher
n für die Zahlen 0, 1 oder 2 steht,
R¹ für gegebenenfalls durch Fluor, Chlor oder Brom substituiertes Methyl, für gegebenenfalls durch Fluor, Chlor und/oder Brom substituiertes Allyl, für gegebenenfalls durch Fluor, Chlor, Brom und/oder Methyl substituiertes Cyclopropyl, für gegebenenfalls durch Fluor, Chlor oder Cyano substituiertes C₁-C₆-Alkoxy oder für C₃-C₄-Alkenyloxy steht,
R² für gegebenenfalls durch Fluor, Chlor, Cyano, Cyclopropyl oder C₁-C₄-Alkoxy substituiertes C₁-C₆-Alkyl, für jeweils gegebenenfalls durch Fluor, Chlor und/oder Brom substituiertes C₃-C₆-Alkenyl oder C₃-C₆-Alkinyl oder für gegebenenfalls durch Fluor, Chlor, Brom und/oder C₁-C₄-Alkyl substituiertes C₃-C₆-Cycloalkyl steht, mit der Maßgabe, daß R² nicht für jeweils unsubstituiertes Methyl oder Ethyl steht, wenn R¹ für unsubstituiertes Methyl und n für O steht, und
R³ für die Gruppierung 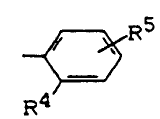 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxycarbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxycarbonyl substituiert ist) steht,
R⁴ und/oder R⁵ weiterhin für Phenyl oder Phenoxy, für C₁-C₄-Alkylcarbonylamino, C₁-C₄-Alkoxycarbonylamino, C₁-C₄-Alkylamino-carbonyl-amino, Di-(C₁-C₄-alkyl)-amino-carbonylamino, oder für den Rest -CO-R⁸ stehen, wobei
R⁸ für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
R³ für den Rest 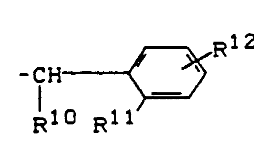 steht, worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
R³ für den Rest 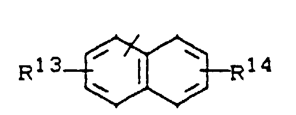 steht, worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
R³ für den Rest 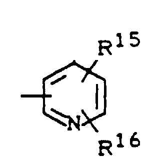 steht, worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), für C₁-C₄-Alkylthio, C₁-C₄-Alkylsulfinyl oder C₁-C₄-Alkylsulfonyl (welche gegebenenfalls durch Fluor und/ oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxycarbonyl oder Dimethylaminocarbonyl stehen;
weiterhin
R³ für den Rest 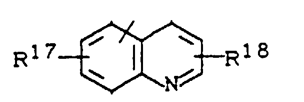 steht, worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)aminosulfonyl stehen;
weiterhin
R³ für den Rest 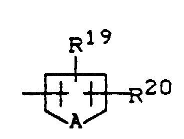 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
R³ für den Rest 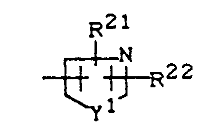 steht, worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
R³ für den Rest 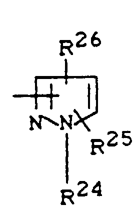 steht, worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht;
weiterhin
R³ für eine der nachstehend aufgeführten Gruppierungen steht, 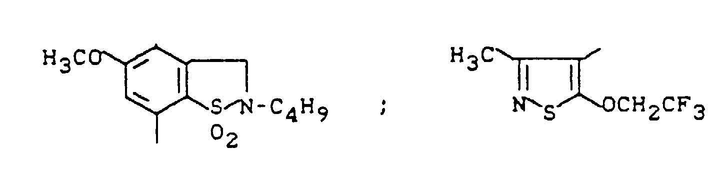 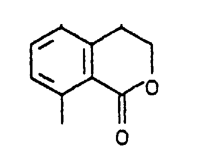 sowie Salze von Verbindungen der Formel (I).

2. Sulfonylaminocarbonyltriazolinone mit über Schwefel gebundenen Substituenten der Formel (I) gemäß Anspruch 1, bei welchen
n für die Zahlen 0, 1 oder 2 steht,
R¹ für gegebenenfalls durch Fluor substituiertes Methyl, für Allyl, für Cyclopropyl, für C₁-C₄-Alkoxy oder für C₃-C₄-Alkenyloxy steht,
R² für gegebenenfalls durch Fluor, Chlor, Methoxy oder Ethoxy substituiertes C₁-C₄-Alkyl, für gegebenenfalls durch Fluor und/oder Chlor substituiertes C₃-C₄-Alkenyl, oder für C₃-C₆-Cycloalkyl steht,
mit der Maßgabe, daß R² nicht für jeweils unsubstituiertes Methyl oder Ethyl steht, wenn R¹ für unsubstituiertes Methyl und n für O steht, und
R³ für die Gruppierung 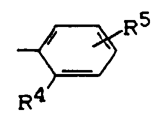 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Methoxyaminosulfonyl, Phenyl, Phenoxy oder C₁-C₃-Alkoxy-carbonyl steht und
R⁵ für Wasserstoff, Fluor, Chlor oder Brom steht;
weiterhin
R³ für den Rest 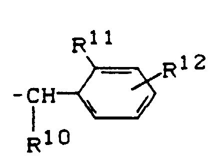 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
R³ für den Rest 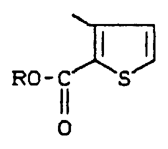 steht,
worin R für C₁-C₄-Alkyl steht, oder
R³ für den Rest 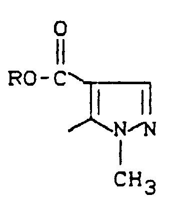 steht,
worin R für C₁-C₄-Alkyl steht,
sowie Salze von Verbindungen der Formel (I).

3. Verfahren zur Herstellung von Sulfonylaminocarbonyltriazolinonen mit über Schwefel gebundenen Substituenten der allgemeinen Formel (I), 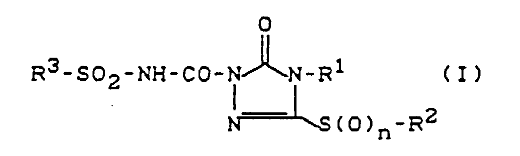 in welcher
n für die Zahlen 0, 1 oder 2 steht,
R¹ für gegebenenfalls durch Fluor, Chlor oder Brom substituiertes Methyl, für gegebenenfalls durch Fluor, Chlor und/oder Brom substituiertes Allyl, für gegebenenfalls durch Fluor, Chlor, Brom und/oder Methyl substituiertes Cyclopropyl, für gegebenenfalls durch Fluor, Chlor oder Cyano substituiertes C₁-C₆-Alkoxy oder für C₃-C₄-Alkenyloxy steht,
R² für gegebenenfalls durch Fluor, Chlor, Cyano, Cyclopropyl oder C₁-C₄-Alkoxy substituiertes C₁-C₆-Alkyl, für jeweils gegebenenfalls durch Fluor, Chlor und/oder Brom substituiertes C₃-C₆-Alkenyl oder C₃-C₆-Alkinyl oder für gegebenenfalls durch Fluor, Chlor, Brom und/oder C₁-C₄-Alkyl substituiertes C₃-C₆-Cycloalkyl steht, mit der Maßgabe, daß R² nicht für jeweils unsubstituiertes Methyl oder Ethyl steht, wenn R¹ für unsubstituiertes Methyl und n für O steht, und
R³ für die Gruppierung 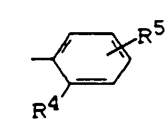 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxycarbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxycarbonyl substituiert ist) steht,
R⁴ und/oder R⁵ weiterhin für Phenyl oder Phenoxy, für C₁-C₄-Alkylcarbonylamino, C₁-C₄-Alkoxycarbonylamino, C₁-C₄-Alkylamino-carbonyl-amino, Di-(C₁-C₄-alkyl)-amino-carbonylamino, oder für den Rest -CO-R⁸ stehen, wobei
R⁸ für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
R³ für den Rest 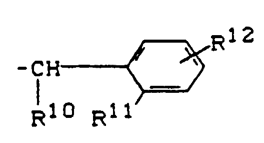 steht, worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
R³ für den Rest 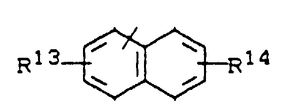 steht, worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
R³ für den Rest 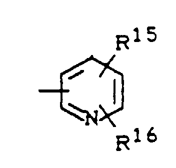 steht, worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxycarbonyl oder Dimethylaminocarbonyl stehen;
weiterhin
R³ für den Rest 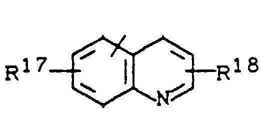 steht, worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)aminosulfonyl stehen;
weiterhin
R³ für den Rest 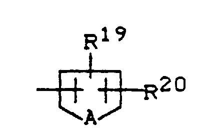 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
R³ für den Rest 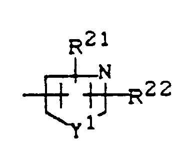 steht, worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
R³ für den Rest 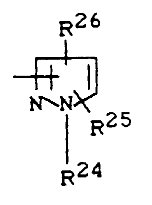 steht, worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht;
weiterhin
R³ für eine der nachstehend aufgeführten Gruppierungen steht, 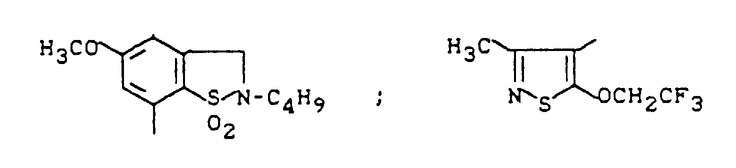 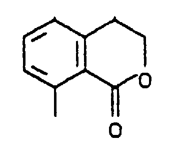
sowie von Salzen der Verbindungen der Formel (I), dadurch gekennzeichnet, daß man
a) Triazolinone der allgemeinen Formel (II) 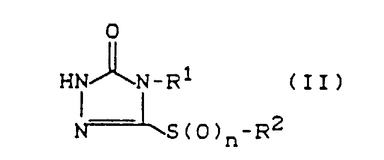 in welcher
n, R¹ und R² die oben (bei Formel I) angegebenen Bedeutungen haben,
mit Sulfonylisocyanaten der allgemeinen Formel (III),
R³-SO₂-N=C=O (III)
in welcher
R³ die oben angegebene Bedeutung hat,
gegebenenfalls in Gegenwart eines Verdünnungs mittels umsetzt, oder daß man
b) Triazolinon-Derivate der allgemeinen Formel (IV) 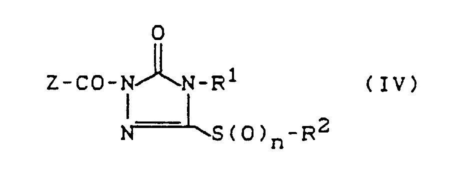 in welcher
n, R¹ und R² die oben angegebenen Bedeutungen haben und
Z für Chlor, C₁-C₄-Alkoxy, Benzyloxy oder Phenoxy steht,
mit Sulfonsäureamiden der allgemeinen Formel (V),
R³-SO₂-NH₂ (V)
in welcher
R³ die oben angegebene Bedeutung hat,
gegebenenfalls in Gegenwart eines Säureakzeptors und gegebenenfalls in Gegenwart eines Verdünnungsmittels umsetzt, oder daß man
c) Triazolinone der allgemeinen Formel (II), 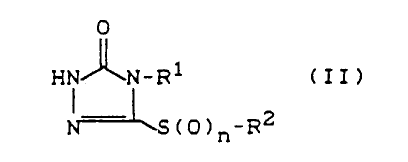 in welcher
n, R¹ und R² die oben angegebenen Bedeutungen haben,
mit Sulfonsäureamid-Derivaten der allgemeinen Formel (VI),
R³-SO₂-NH-CO-Z (VI)
in welcher
R³ die oben angegebene Bedeutung hat und
Z für Chlor, C₁-C₄-Alkoxy, Benzyloxy oder Phenoxy steht,
gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

4. Herbizide Mittel, gekennzeichnet durch einen Gehalt an mindestens einem Sulfonylaminocarbonyltriazolinon der Formel (I) gemäß Anspruch 1 oder 3.

5. Verwendung von Sulfonylaminocarbonyltriazolinonen der Formel (I) gemäß Anspruch 1 oder 3 zur Bekämpfung von unerwünschtem Pflanzenwachstum.

6. Verfahren zur Bekämpfung von Unkräutern, dadurch gekennzeichnet, daß man Sulfonylaminocarbonyltriazolinone der Formel (I) gemäß Anspruch 1 oder 3 auf die Unkräuter oder ihren Lebensraum einwirken läßt.

7. Verfahren zur Herstellung von herbiziden Mitteln, dadurch gekennzeichnet, daß man Sulfonylaminocarbonyltriazolinone der Formel (I) gemäß Anspruch 1 oder 3 mit Streckmitteln und/oder oberflächenaktiven Mitteln vermischt.

## Claims

1. Sulphonylaminocarbonyltriazolinones having sulphur-bonded substituents of the general formula (I) 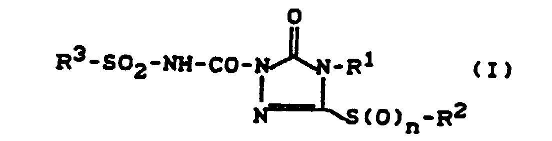 in which
n represents the numbers 0, 1 or 2,
R¹ represents optionally fluorine-, chlorine- or bromine-substituted methyl, represents optionally fluorine-, chlorine- and/or bromine-substituted allyl, represents optionally fluorine-, chlorine-, bromine- and/or methyl-substituted cyclopropyl, represents optionally fluorine-, chlorine- or cyano-substituted C₁-C₆-alkoxy or represents C₃-C₄-alkenyloxy,
R² represents optionally fluorine-, chlorine-, cyano-, cyclopropyl- or C₁-C₄-alkoxy-substituted C₁-C₆-alkyl, represents respectively optionally fluorine-, chlorine- and/or bromine-substituted C₃-C₆-alkenyl, or C₃-C₆-alkinyl or represents optionally fluorine-, chlorine-, bromine- and/or C₁-C₄-alkyl-substituted C₃-C₆-cycloalkyl, with the proviso that R² does not represent respectively unsubstituted methyl or ethyl if R¹ represents unsubstituted methyl and n represents 0, and
R³ represents the grouping 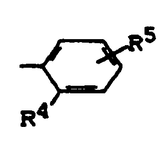 in which
R⁴ and R⁵ are identical or different and each represent hydrogen, fluorine, chlorine, bromine, iodine, nitro, C₁-C₆-alkyl (which is optionally substituted by fluorine, chlorine, bromine, cyano, carboxyl, C₁-C₄-alkoxycarbonyl, C₁-C₄-alkylaminocarbonyl, di-(C₁-C₄-alkyl)amino-carbonyl, hydroxyl, C₁-C₄-alkoxy, formyloxy, C₁-C₄-alkylcarbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)-aminosulphonyl, C₃-C₆-cycloalkyl or phenyl), represents C₂-C₆-alkenyl (which is optionally substituted by fluorine, chlorine, bromine, cyano, C₁-C₄-alkoxy-carbonyl, carboxyl or phenyl), represents C₂-C₆-alkinyl (which is optionally substituted by fluorine, chlorine, bromine, cyano, C₁-C₄-alkoxy-carbonyl, carboxyl or phenyl), represents C₁-C₄-alkoxy (which is optionally substituted by fluorine, chlorine, bromine, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), represents C₁-C₄-alkylthio (which is optionally substituted by fluorine, chlorine, bromine, cyano, carboxyl, C₁-C₄-alkoxy-carbonyl, C₁-C₄-alkylthio, C₁-C₄-alkylsulphinyl, or C₁-C₄-alkylsulphonyl), represents C₃-C₆-alkenyloxy (which is optionally substituted by fluorine, chlorine, bromine, cyano or C₁-C₄-alkoxy-carbonyl), represents C₂-C₆-alkenylthio (which is optionally substituted by fluorine, chlorine, bromine, cyano, nitro, C₁-C₃-alkylthio or C₁-C₄-alkoxycarbonyl), C₃-C₆-alkinyloxy, C₃-C₆-alkinylthio or represents the radical -S(O)ₚ-R⁶ where
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino, phenyl or represents the radical -NHOR⁷ where
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-alkylsulphonyl, C₁-C₄-alkylcarbonyl, C₁-C₄-alkoxy-carbonyl, C₁-C₄-alkylamino-carbonyl or di-(C₁-C₄-alkyl)-amino-carbonyl), represents C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl, phenyl-C₁-C₂-alkyl (which is optionally substituted by fluorine, chlorine, nitro, cyano, C₁-C₄-alkyl, C₁-C₄-alkoxy or C₁-C₄-alkoxycarbonyl), represents benzhydryl or represents phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxycarbonyl),
R⁴ and/or R⁵ furthermore represent phenyl or phenoxy, represent C₁-C₄-alkylcarbonylamino, C₁-C₄-alkoxycarbonylamino, C₁-C₄-alkylaminocarbonyl-amino, di-(C₁-C₄-alkyl)-amino-carbonylamino, or represent the radical -CO-R⁸ where
R⁸ represents C₁-C₆-alkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino (which are optionally substituted by fluorine and/or chlorine),
R⁴ and/or R⁵ furthermore represent trimethylsilyl, thiazolinyl, C₁-C₄-alkylsulphonyloxy, di-(C₁-C₄-alkyl)-aminosulphonylamino or represent the radical -CH=N-R⁹ where
R⁹ represents optionally fluorine-, chlorine-, cyano-, carboxyl-, C₁-C₄-alkoxy-, C₁-C₄-alkylthio-, C₁-C₄-alkylsulphinyl- or C₁-C₄-alkylsulphonyl-substituted C₁-C₆-alkyl, represents optionally fluorine- or chlorine-substituted benzyl, represents optionally fluorine- or chlorine-substituted C₃-C₆-alkenyl or C₃-C₆-alkinyl, represents optionally fluorine-, chlorine-, bromine-, C₁-C₄-alkyl-, C₁-C₄-alkoxy-, trifluoromethyl-, trifluoromethoxy- or trifluoromethylthio-substituted phenyl, represents optionally fluorine-and/or chlorine-substituted C₁-C₆-alkoxy, C₃-C₆-alkenoxy, C₃-C₆-alkinoxy or benzyloxy, represents amino, C₁-C₄-alkylamino, di-(C₁-C₄-alky)-amino, phenylamino, C₁-C₄-alkyl-carbonyl-amino, C₁-C₄-alkoxy-carbonylamino, C₁-C₄-alkyl-sulphonylamino or represents optionally fluorine-, chlorine-, bromine- or methyl-substituted phenylsulphonylamino,
furthermore
R³ represents the radical 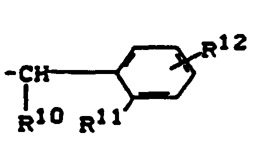 where
R¹⁰ represents hydrogen or C₁-C₄-alkyl,
R¹¹ and R¹² are identical or different and each represents hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), carboxyl, C₁-C₄-alkoxy-carbonyl, dimethylaminocarbonyl, C₁-C₄-alkylsulphonyl or di-(C₁-C₄-alkyl)-aminosulphonyl;
furthermore
R³ represents the radical 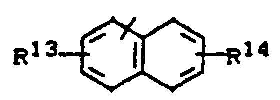 where
R¹³ and R¹⁴ are identical or different and each represents hydrogen, fluorine, chlorine, bromine, nitro, cyano, C₁-C₄-alkyl (which is optionally substituted by fluorine and/or chlorine) or C₁-C₄-alkoxy (which is optionally substituted by fluorine and/or chlorine);
furthermore
R³ represents the radical 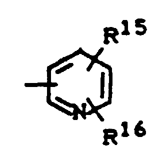 where
R¹⁵ and R¹⁶ are identical or different and each represents hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), represents C₁-C₄-alkylthio, C₁-C₄-alkylsulphinyl or C₁-C₄-alkylsulphonyl (which are optionally substituted by fluorine and/or chlorine), represents aminosulphonyl, mono-(C₁-C₄-alkyl)-aminosulphonyl, represents di-(C₁-C₄-alkyl)aminosulphonyl, or C₁-C₄-alkoxy-carbonyl or dimethylaminocarbonyl;
furthermore
R³ represents the radical 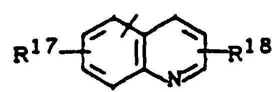 where
R¹⁷ and R¹⁸ are identical or different and each represents hydrogen, fluorine, chlorine, bromine, C₁-C₄-alkyl (which is optionally substituted by fluorine and/or bromine), C₁-C₄-alkoxy (which is optionally substituted by fluorine and/or chlorine), represents C₁-C₄-alkylthio, C₁-C₄-alkylsulphinyl or C₁-C₄-alkylsulphonyl (which are optionally substituted by fluorine and/or chlorine), or represents di-(C₁-C₄-alkyl)-aminosulphonyl;
furthermore
R³ represents the radical 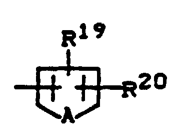 where
R¹⁹ and R²⁰ are identical or different and each represents hydrogen, fluorine, chlorine, bromine, cyano, nitro, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (which is optionally substituted by fluorine and/or chlorine), di-(C₁-C₄-alkyl)-amino-sulphonyl, C₁-C₄-alkoxy-carbonyl or dimethylaminocarbonyl, and
A represents oxygen, sulphur or the grouping N-Z¹ where
Z¹ represents hydrogen, C₁-C₄-alkyl (which is optionally substituted by fluorine, chlorine, bromine or cyano), C₃-C₆-cycloalkyl, benzyl, phenyl (which is optionally substituted by fluorine, chlorine, bromine or nitro), C₁-C₄-alkylcarbonyl, C₁-C₄-alkoxy-carbonyl or di- (C₁-C₄-alkyl)-aminocarbonyl;
furthermore
R³ represents the radical 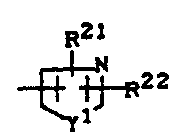 where
R²¹ and R²² are identical or different and each represents hydrogen, C₁-C₄-alkyl, halogen, C₁-C₄-alkoxycarbonyl, C₁-C₄-alkoxy or C₁-C₄-halogenoalkoxy,
Y¹ represents sulphur or the grouping N-R²³ where
R²³ represents hydrogen or C₁-C₄-alkyl;
furthermore
R³ represents the radical 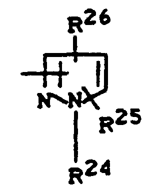 where
R²⁴ represents hydrogen, C₁-C₄-alkyl, benzyl, pyridyl, quinolinyl or phenyl,
R²⁵ represents hydrogen, halogen, cyano, nitro, C₁-C₄-alkyl (which is optionally substituted by fluorine and/or chlorine), C₁-C₄-alkoxy (which is optionally substituted by fluorine and/or chlorine), dioxolanyl or C₁-C₄-alkoxy-carbonyl and
R²⁶ represents hydrogen, halogen or C₁-C₄-alkyl;
furthermore
R³ represents one of the groupings listed below 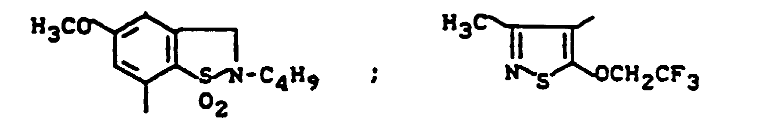 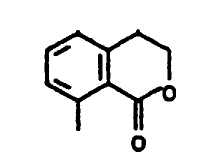
and salts of compounds of the formula (I).

2. Sulphonylaminocarbonyltriazolinones having sulphur-bonded substituents of the formula (I) according to Claim 1 where
n represents the numbers 0, 1 or 2,
R¹ represents optionally fluorine-substituted methyl, represents allyl, represents cyclopropyl, represents C₁-C₄-alkoxy or represents C₃-C₄-alkenyloxy,
R² represents optionally fluorine-, chlorine-, methoxy- or ethoxy-substituted C₁-C₄-alkyl, represents optionally fluorine- and/or chlorine-substituted C₃-C₄-alkenyl or represents C₃-C₆-cycloalkyl,
with the proviso that R² does not represent respectively unsubstituted methyl or ethyl if R¹ represents unsubstituted methyl and n represents 0, and
R³ represents the grouping 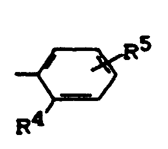 where
R⁴ represents fluorine, chlorine, bromine, methyl, trifluoromethyl, methoxy, difluoromethoxy, trifluoromethoxy, 2-chloro-ethoxy, 2-methoxy-ethoxy, C₁-C₃-alkylthio, C₁-C₃-alkylsulphinyl, C₁-C₃-alkylsulphonyl, dimethylaminosulphonyl, diethylaminosulphonyl, N-methoxy-N-methylaminosulphonyl, methoxyaminosulphonyl, phenyl, phenoxy or C₁-C₃-alkoxy-carbonyl and
R⁵ represents hydrogen, fluorine, chlorine or bromine;
furthermore
R³ represents the radical 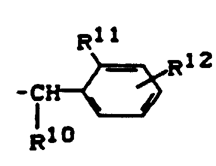 where
R¹⁰ represents hydrogen,
R¹¹ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
R¹² represents hydrogen;
furthermore
R³ represents the radical 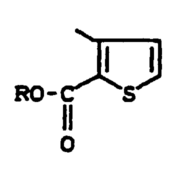 where R represents C₁-C₄-alkyl, or
R³ represents the radical 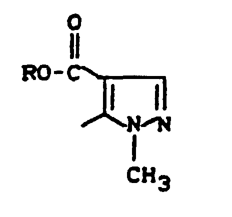 where R represents C₁-C₄-alkyl,
and salts of compounds of the formula (I).

3. Process for preparing sulphonylaminocarbonyltriazolinones having sulphur-bonded substituents of the general formula (I) 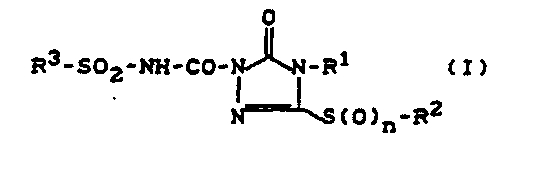 in which
n represents the numbers 0, 1 or 2,
R¹ represents optionally fluorine-, chlorine- or bromine-substituted methyl, represents optionally fluorine-, chlorine- and/or bromine-substituted allyl, represents optionally fluorine-, chlorine-, bromine- and/or methyl-substituted cyclopropyl, represents optionally fluorine-, chlorine- or cyano-substituted C₁-C₆-alkoxy or represents C₃-C₄-alkenyloxy,
R² represents optionally fluorine-, chlorine-, cyano-, cyclopropyl- or C₁-C₄-alkoxy-substituted C₁-C₆-alkyl, represents respectively optionally fluorine-, chlorine- and/or bromine-substituted C₃-C₆-alkenyl, or C₃-C₆-alkinyl or represents optionally fluorine-, chlorine-, bromine- and/or C₁-C₄-alkyl-substituted C₃-C₆-cycloalkyl, with the proviso that R² does not represent respectively unsubstituted methyl or ethyl if R¹ represents unsubstituted methyl and n represents 0, and
R³ represents the grouping 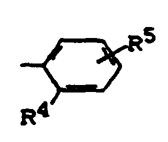 in which
R⁴ and R⁵ are identical or different and each represent hydrogen, fluorine, chlorine, bromine, iodine, nitro, C₁-C₆-alkyl (which is optionally substituted by fluorine, chlorine, bromine, cyano, carboxyl, C₁-C₄-alkoxycarbonyl, C₁-C₄-alkylaminocarbonyl, di-(C₁-C₄-alkyl)amino-carbonyl, hydroxyl, C₁-C₄-alkoxy, formyloxy, C₁-C₄-alkylcarbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄- alkyl)-aminosulphonyl, C₃-C₆-cycloalkyl or phenyl), represents C₂-C₆-alkenyl (which is optionally substituted by fluorine, chlorine, bromine, cyano, C₁-C₄-alkoxy-carbonyl, carboxyl or phenyl), represents C₂-C₆-alkinyl (which is optionally substituted by fluorine, chlorine, bromine, cyano, C₁-C₄-alkoxy-carbonyl, carboxyl or phenyl), represents C₁-C₄-alkoxy (which is optionally substituted by fluorine, chlorine, bromine, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), represents C₁-C₄-alkylthio (which is optionally substituted by fluorine, chlorine, bromine, cyano, carboxyl, C₁-C₄-alkoxy-carbonyl, C₁-C₄-alkylthio, C₁-C₄-alkylsulphinyl, or C₁-C₄-alkylsulphonyl), represents C₃-C₆-alkenyloxy (which is optionally substituted by fluorine, chlorine, bromine, cyano or C₁-C₄-alkoxy-carbonyl), represents C₂-C₆-alkenylthio (which is optionally substituted by fluorine, chlorine, bromine, cyano, nitro, C₁-C₃-alkylthio or C₁-C₄-alkoxycarbonyl), C₃-C₆-alkinyloxy, C₃-C₆-alkinylthio or represents the radical -S(O)ₚ-R⁶ where
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino, phenyl or represents the radical -NHOR⁷ where
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-alkylsulphonyl, C₁-C₄-alkylcarbonyl, C₁-C₄-alkoxy-carbonyl, C₁-C₄-alkylamino-carbonyl or di-(C₁-C₄-alkyl)-amino-carbonyl), represents C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl, phenyl-C₁-C₂-alkyl (which is optionally substituted by fluorine, chlorine, nitro, cyano, C₁-C₄-alkyl, C₁-C₄-alkoxy or C₁-C₄-alkoxycarbonyl), represents benzhydryl or represents phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxycarbonyl),
R⁴ and/or R⁵ furthermore represent phenyl or phenoxy, represent C₁-C₄-alkylcarbonylamino, C₁-C₄-alkoxycarbonylamino, C₁-C₄-alkylamino-carbonylamino, di-(C₁-C₄-alkyl)-amino-carbonylamino, or represent the radical -CO-R⁸ where
R⁸ represents C₁-C₆-alkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino (which are optionally substituted by fluorine and/or chlorine),
R⁴ and/or R⁵ furthermore represent trimethylsilyl, thiazolinyl, C₁-C₄-alkylsulphonyloxy, di-(C₁-C₄-alkyl)-aminosulphonylamino or represent the radical -CH=N-R⁹ where
R⁹ represents optionally fluorine-, chlorine-, cyano-, carboxyl-, C₁-C₄-alkoxy-, C₁-C₄-alkylthio-, C₁-C₄-alkylsulphinyl- or C₁-C₄-alkylsulphonyl-substituted C₁-C₆-alkyl, represents optionally fluorine- or chlorine-substituted benzyl, represents optionally fluorine- or chlorine-substituted C₃-C₆-alkenyl or C₃-C₆-alkinyl, represents optionally fluorine-, chlorine-, bromine-, C₁-C₄-alkyl-, C₁-C₄-alkoxy-, trifluoromethyl-, trifluoromethoxy- or trifluoromethylthio-substituted phenyl, represents optionally fluorine- and/or chlorine-substituted C₁-C₆-alkoxy, C₃-C₆-alkenoxy, C₃-C₆-alkinoxy or benzyloxy, represents amino, C₁-C₄-alkylamino, di-(C₁-C₄-alkyl)-amino, phenylamino, C₁-C₄-alkyl-carbonyl-amino, C₁-C₄-alkoxy-carbonylamino, C₁-C₄-alkyl-sulphonylamino or represents optionally fluorine-, chlorine-, bromine- or methyl-substituted phenylsulphonylamino,
furthermore
R³ represents the radical 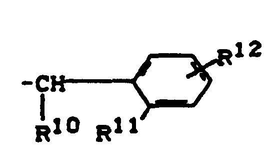 where
R¹⁰ represents hydrogen or C₁-C₄-alkyl,
R¹¹ and R¹² are identical or different and each represents hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), carboxyl, C₁-C₄-alkoxy-carbonyl, dimethylaminocarbonyl, C₁-C₄-alkylsulphonyl or di-(C₁-C₄-alkyl)-aminosulphonyl;
furthermore
R³ represents the radical 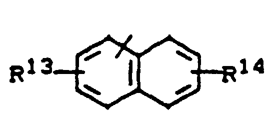 where
R¹³ and R¹⁴ are identical or different and each represents hydrogen, fluorine, chlorine, bromine, nitro, cyano, C₁-C₄-alkyl (which is optionally substituted by fluorine and/or chlorine) or C₁-C₄-alkoxy (which is optionally substituted by fluorine and/or chlorine);
furthermore
R³ represents the radical 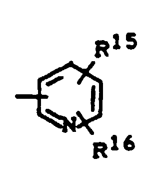 where
R¹⁵ and R¹⁶ are identical or different and each represents hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), represents C₁-C₄-alkylthio, C₁-C₄-alkylsulphinyl or C₁-C₄-alkylsulphonyl (which are optionally substituted by fluorine and/or chlorine), represents aminosulphonyl, mono-(C₁-C₄-alkyl)-aminosulphonyl, represents di-(C₁-C₄-alkyl)-aminosulphonyl, or C₁-C₄-alkoxy-carbonyl or dimethylaminocarbonyl;
furthermore
R³ represents the radical 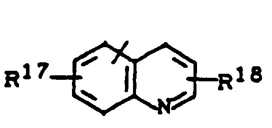 where
R¹⁷ and R¹⁸ are identical or different and each represents hydrogen, fluorine, chlorine, bromine, C₁-C₄-alkyl (which is optionally substituted by fluorine and/or bromine), C₁-C₄-alkoxy (which is optionally substituted by fluorine and/or chlorine), represents C₁-C₄-alkylthio, C₁-C₄-alkylsulphinyl or C₁-C₄-alkylsulphonyl (which are optionally substituted by fluorine and/or chlorine), or represents di-(C₁-C₄-alkyl)-aminosulphonyl;
furthermore
R³ represents the radical 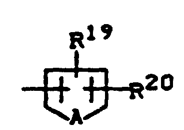 where
R¹⁹ and R²⁰ are identical or different and each represents hydrogen, fluorine, chlorine, bromine. cyano, nitro C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (which is optionally substituted by fluorine and/or chlorine), di-(C₁-C₄-alkyl)amino-sulphonyl, C₁-C₄-alkoxy-carbonyl or dimethylaminocarbonyl, and
A represents oxygen, sulphur or the grouping N-Z¹ where
Z¹ represents hydrogen, C₁-C₄-alkyl (which is optionally substituted by fluorine, chlorine, bromine or cyano), C₃-C₆-cycloalkyl, benzyl, phenyl (which is optionally substituted by fluorine, chlorine, bromine or nitro), C₁-C₄-alkylcarbonyl, C₁-C₄-alkoxy-carbonyl or di-(C₁-C₄-alky)-aminocarbonyl;
furthermore
R³ represents the radical 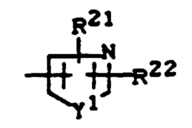 where
R²¹ and R²² are identical or different and each represents hydrogen, C₁-C₄-alkyl, halogen, C₁-C₄-alkoxycarbonyl, C₁-C₄-alkoxy or C₁-C₄-halogenoalkoxy,
Y¹ represents sulphur or the grouping N-R²³ where
R²³ represents hydrogen or C₁-C₄-alkyl;
furthermore
R³ represents the radical 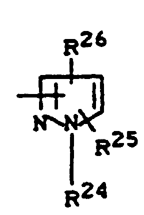 where
R²⁴ represents hydrogen, C₁-C₄-alkyl, benzyl, pyridyl, quinolinyl or phenyl,
R²⁵ represents hydrogen, halogen, cyano, nitro, C₁-C₄-alkyl (which is optionally substituted by fluorine and/or chlorine), C₁-C₄-alkoxy (which is optionally substituted by fluorine and/or chlorine), dioxolanyl or C₁-C₄-alkoxy-carbonyl and
R²⁶ represents hydrogen, halogen or C₁-C₄-alkyl;
furthermore
R³ represents one of the groupings listed below 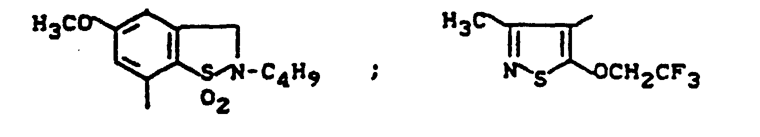 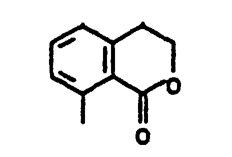 and salts of compounds of the formula (I), characterized in that
a) triazolinones of the general formula (II) 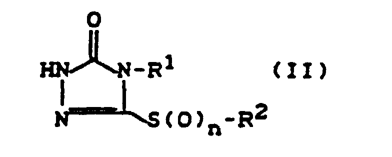 in which
n, R¹ and R² are each as defined above (under Formula I)
are reacted with sulphonylisocyanates of the general formula (III)
R³-SO₂-N=C=O (III)
in which
R³ is as defined above,
if appropriate in the presence of a diluent, or in that
b) triazolinone derivatives of the general formula (IV) 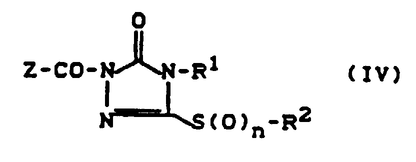 in which
n, R¹ and R² are each as defined above and
Z represents chlorine, C₁-C₄-alkoxy, benzyloxy or phenoxy,
are reacted with sulphonamides of the general formula (V)
R³-SO₂-NH₂ (V)
in which
R³ is as defined above,
if appropriate in the presence of an acid acceptor and if appropriate in the presence of a diluent, or in that
c) triazolinones of the general formula (II) 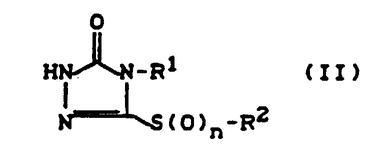 in which
n, R¹ and R² are each as defined above
are reacted with sulphonamide derivatives of the general formula (VI)
R³-SO₂-NH-CO-Z (VI)
in which
R³ is as defined above and
Z represents chlorine, C₁-C₄-alkoxy, benzyloxy or phenoxy,
if appropriate in the presence of an acid acceptor and if appropriate in the presence of a diluent and, if required, salts are prepared by customary methods from the compounds of the formula (I) prepared by processes (a), (b) or (c).

4. Herbicidal compositions, characterized in that they contain at least one sulphonylaminocarbonyltriazolinone of the formula (I) according to Claim 1 or 3.

5. Use of sulphonylaminocarbonyltriazolinones of the formula (I) according to Claim 1 or 3 for controlling unwanted vegetation.

6. Method for controlling weeds, characterized in that sulphonylaminocarbonyltriazolinones of the formula (I) according to Claim 1 or 3 are allowed to act on the weeds or their habitat.

7. Process for preparing herbicidal compositions, characterized in that sulphonylaminocarbonyltriazolinones of the formula (I) according to Claim 1 or 3 are mixed with extenders and/or surfactants.

## Revendications

1. Sulfonylaminocarbonyltriazolinones portant des substituants liés via un atome de soufre, répondant à la formule générale (I) 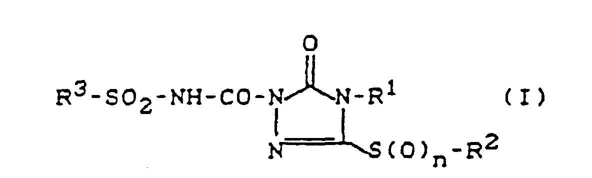 dans laquelle
n représente les nombres 0, 1 ou 2,
R¹ représente un groupe méthyle portant éventuellement un ou plusieurs substituants identiques ou différents fluoro, chloro ou bromo; un groupe allyle portant éventuellement un ou plusieurs substituants identiques ou différents fluoro, chloro et/ou bromo; un groupe cyclopropyle portant éventuellement un ou plusieurs substituants identiques ou différents fluoro, chloro, bromo et/ou méthyle; un groupe alcoxy en C₁-C₆ portant éventuellement un ou plusieurs substituants identiques ou différents fluoro, chloro ou cyano; ou encore un groupe alcényl(en C₃-C₄)oxy,
R² représente un groupe alkyle en C₁-C₆ portant éventuellement un ou plusieurs substituants identiques ou différents fluoro, chloro, cyano, cyclopropyle ou alcoxy en C₁-C₄; un groupe alcényle en C₃-C₆ ou un groupe alcynyle en C₃-C₆ portant respectivement le cas échéant un ou plusieurs substituants identiques ou différents fluoro, chloro et/ou bromo; ou représente un groupe cycloalkyle en C₃-C₆ portant éventuellement un ou plusieurs substituants identiques ou différents fluoro, chloro, bromo et/ou alkyle en C₁-C₄; avec cette mesure que R² ne représente pas un groupe méthyle ou un groupe éthyle respectivement non substitués lorsque R¹ représente un groupe méthyle non substitué et n représente 0, et
R³ représente le groupement 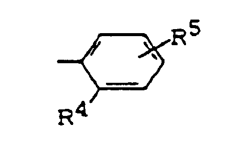 dans lequel
R⁴ et R⁵ sont identiques ou différents et représentent un atome d'hydrogène, un atome de fluor, un atome de chlore, un atome de brome, un atome d'iode, un groupe nitro, un groupe alkyle en C₁-C₆ (qui porte éventuellement un ou plusieurs substituants identiques ou différents fluoro, chloro, bromo, cyano, carboxyle, alcoxy(en C₁-C₄)carbonyle, alkyl(en C₁-C₄)aminocarbonyle, di-(alkyl en C₁-C₄)-aminocarbonyle, hydroxyle, alcoxy en C₁-C₄, formyloxy, alkyl(en C₁-C₄)-carbonyloxy, alcoxy(en C₁-C₄)carbonyloxy, alkyl(en C₁-C₄) aminocarbonyloxy, alkyl(en C₁-C₄)thio, alkyl(en C₁-C₄)sulfinyle, alkyl(en C₁-C₄)sulfonyle, di-(alkyl en C₁-C₄)aminosulfonyle, cycloalkyle en C₃-C₆ ou phényle); un groupe alcényle en C₂-C₆ (qui porte éventuellement un ou plusieurs substituants identiques ou différents fluoro, chloro, bromo, cyano, alcoxy(en C₁-C₄)carbonyle, carboxyle ou phényle); un groupe alcynyle en C₂-C₆ (qui porte éventuellement un ou plusieurs substituants identiques ou différents fluoro, chloro, bromo, cyano, alcoxy(en C₁-C₄)carbonyle, carboxyle ou phényle); un groupe alcoxy en C₁-C₄ (qui porte éventuellement un ou plusieurs substituants identiques ou différents fluoro, chloro, bromo, cyano, carboxyle, alcoxy(en C₁-C₄)carbonyle, alcoxy en C₁-C₄, alkyl(en C₁-C₄)thio, alkyl(en C₁-C₄)-sulfinyle ou alkyl(en C₁-C₄) sulfonyle) ; un groupe alkyl(en C₁-C₄)thio (qui porte éventuellement un ou plusieurs substituants identiques ou différents fluoro, chloro, bromo, cyano, carboxyle, alcoxy(en C₁-C₄)carbonyle, alkyl(en C₁-C₄)thio, alkyl(en C₁-C₄)sulfinyle ou alkyl(en C₁-C₄)sulfonyle); un groupe alcényl(en C₃-C₆)oxy (qui porte éventuellement un ou plusieurs substituants identiques ou différents fluoro, chloro, bromo, cyano ou alcoxy(en C₁-C₄)carbonyle); un groupe alcényl(en C₂-C₆)thio (qui porte éventuellement un ou plusieurs substituants identiques ou différents fluoro, chloro, bromo, cyano, nitro, alkyl(en C₁-C₃)thio ou alcoxy(en C₁-C₄)carbonyle); un groupe alcynyl(en C₃-C₆)oxy; un groupe alcynyl(en C₃-C₆)thio, ou encore représente le radical -S(O)ₚ-R⁶ où
p représente les nombres 1 ou 2, et
R⁶ représente un groupe alkyle en C₁-C₄ (qui porte éventuellement un ou plusieurs substituants identiques ou différents fluoro, chloro, bromo, cyano ou alcoxy(en C₁-C₄)carbonyle); un groupe alcényle en C₃-C₆, un groupe alcynyle en C₃-C₆, un groupe alcoxy en C₁-C₄, un groupe alcoxy(en C₁-C₄)alkyl (en C₁-C₄)amino, un groupe alkyl(en C₁-C₄)amino, un groupe di-(alkyl en C₁-C₄)amino, un groupe phényle ou encore représente le radical -NHOR⁷ où
R⁷ représente un groupe alkyle en C₁-C₁₂ (qui porte éventuellement un ou plusieurs substituants identiques ou différents fluoro, chloro, cyano, alcoxy en C₁-C₄, alkyl(en C₁-C₄)thio, alkyl(en C₁-C₄)sulfinyle, alkyl(en C₁-C₄)sulfonyle, alkyl(en C₁-C₄)-carbonyle, alcoxy(en C₁-C₄)carbonyle, alkyl(en C₁-C₄)-aminocarbonyle ou di-(alkyl en C₁-C₄)aminocarbonyle); un groupe alcényle en C₃-C₆ (qui porte éventuellement un ou plusieurs substituants identiques ou différents fluoro, chloro ou bromo); un groupe alcynyle en C₃-C₆; un groupe cycloalkyle en C₃-C₆; un groupe cycloalkyl(en C₃-C₆)-alkyle en C₁-C₂; un groupe phénylalkyle en C₁-C₂ (qui porte éventuellement un ou plusieurs substituants identiques ou différents fluoro, chloro, nitro, cyano, alkyle en C₁-C₄, alcoxy en C₁-C₄ ou alcoxy(en C₁-C₄)-carbonyle); un groupe benzhydryle; ou encore un groupe phényle (qui porte éventuellement un ou plusieurs substituants identiques ou différents fluoro, chloro, nitro, cyano, alkyle en C₁-C₄, trifluorométhyle, alcoxy en C₁-C₄, fluoroalcoxy en C₁-C₂, alkyl(en C₁-C₄)thio, trifluorométhylthio ou alcoxy(en C₁-C₄)carbonyle),
R⁴ et/ou R⁵ représentent, en outre, un groupe phényle ou un groupe phénoxy; un groupe alkyl(en C₁-C₄)carbonylamino, un groupe alcoxy(en C₁-C₄) carbonylamino, un groupe alkyl(en C₁-C₄)aminocarbonylamino, un groupe di-(alkyl en C₁-C₄)aminocarbonylamino; ou représentent le radical -CO-R⁸ où
R⁸ représente un groupe alkyle en C₁-C₆, un groupe alcoxy en C₁-C₆, un groupe cycloalcoxy en C₃-C₆, un groupe alcényl(en C₃-C₆)oxy, un groupe alkyl(en C₁-C₄)thio, un groupe alkyl(en C₁-C₄)amino, un groupe alcoxy(en C₁-C₄)amino, un groupe alcoxy(en C₁-C₄)alkyl(en C₁-C₄)amino ou un groupe di-(alkyl en C₁-C₄)amino (qui portent éventuellement un ou plusieurs substituants identiques ou différents fluoro et/ou chloro),
R⁴ et/ou R⁵ représentent, en outre, un groupe triméthylsilyle, un groupe thiazolinyle, un groupe alkyl(en C₁-C₄)sulfonyloxy, un groupe di-(alkyl en C₁-C₄)aminosulfonylamino ou représentent le radical -CH=N-R⁹ où
R⁹ représente un groupe alkyle en C₁-C₆ portant éventuellement un ou plusieurs substituants identiques ou différents fluoro, chloro, cyano, carboxyle, alcoxy en C₁-C₄, alkyl(en C₁-C₄)thio, alkyl(en C₁-C₄)sulfinyle ou alkyl(en C₁-C₄)sulfonyle; un groupe benzyle portant éventuellement un ou plusieurs substituants identiques ou différents fluoro ou chloro; un groupe alcényle en C₃-C₆ ou un groupe alcynyle en C₃-C₆ portant éventuellement un ou plusieurs substituants identiques ou différents fluoro ou chloro; un groupe phényle portant éventuellement un ou plusieurs substituants identiques ou différents fluoro, chloro, bromo, alkyle en C₁-C₄, alcoxy en C₁-C₄, trifluorométhyle, trifluorométhoxy ou trifluorométhylthio; un groupe alcoxy en C₁-C₆, un groupe alcénoxy en C₃-C₆, un groupe alcynoxy en C₃-C₆ ou un groupe benzyloxy, chacun de ces groupes portant éventuellement un ou plusieurs substituants identiques ou différents fluoro et/ou chloro; un groupe amino, un groupe alkyl(en C₁-C₄)amino, un groupe di-(alkyl en C₁-C₄)amino, un groupe phénylamino, un groupe alkyl(en C₁-C₄)carbonylamino, un groupe alcoxy(en C₁-C₄)carbonylamino, un groupe alkyl(en C₁-C₄)sulfonylamino; ou représente un groupe phénylsulfonylamino portant éventuellement un ou plusieurs substituants identiques ou différents fluoro, chloro, bromo ou méthyle,
en outre,
R³ représente le radical 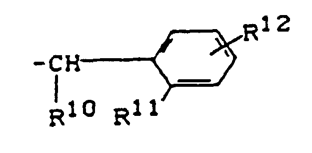 dans lequel
R¹⁰ représente un atome d'hydrogène ou un groupe alkyle en C₁-C₄,
R¹¹ et R¹² sont identiques ou différents et représentent un atome d'hydrogène, un atome de fluor, un atome de chlore, un atome de brome, un groupe nitro, un groupe cyano, un groupe alkyle en C₁-C₄ (qui porte éventuellement un ou plusieurs substituants identiques ou différents fluoro et/ou chloro); un groupe alcoxy en C₁-C₄ (qui porte éventuellement un ou plusieurs substituants identiques ou différents fluoro et/ou chloro); un groupe carboxyle, un groupe alcoxy(en C₁-C₄)carbonyle, un groupe diméthylaminocarbonyle, un groupe alkyl(en C₁-C₄)sulfonyle ou un groupe di-(alkyl en C₁-C₄)aminosulfonyle;
en outre,
R³ représente le radical 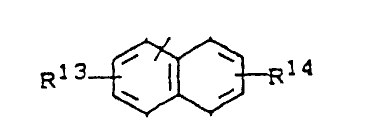 dans lequel
R¹³ et R¹⁴ sont identiques ou différents et représentent un atome d'hydrogène, un atome de fluor, un atome de chlore, un atome de brome, un groupe nitro, un groupe cyano, un groupe alkyle en C₁-C₄ (qui porte éventuellement un ou plusieurs substituants identiques ou différents fluoro et/ou chloro) ou un groupe alcoxy en C₁-C₄ (qui porte éventuellement un ou plusieurs substituants identiques ou différents fluoro et/ou chloro);
en outre,
R³ représente le radical 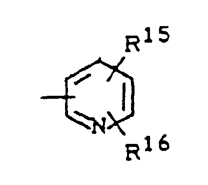 dans lequel
R¹⁵ et R¹⁶ sont identiques ou différents et représentent un atome d'hydrogène, un atome de fluor, un atome de chlore, un atome de brome, un groupe nitro, un groupe cyano, un groupe alkyle en C₁-C₄ (qui porte éventuellement un ou plusieurs substituants identiques ou différents fluoro et/ou chloro), un groupe alcoxy en C₁-C₄ (qui porte éventuellement un ou plusieurs substituants identiques ou différents fluoro et/ou chloro); un groupe alkyl(en C₁-C₄)thio, un groupe alkyl(en C₁-C₄)sulfinyle ou un groupe alkyl(en C₁-C₄)sulfonyle (qui portent éventuellement un ou plusieurs substituants identiques ou différents fluoro et/ou chloro); un groupe aminosulfonyle, un groupe mono(alkyl en C₁-C₄)aminosulfonyle; un groupe di-(alkyl en C₁-C₄)aminosulfonyle ou un groupe alcoxy(en C₁-C₄)carbonyle ou encore un groupe diméthylaminocarbonyle;
en outre,
R³ représente le radical 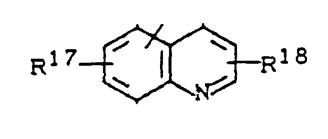 dans lequel
R¹⁷ et R¹⁸ sont identiques ou différents et représentent un atome d'hydrogène, un atome de fluor, un atome de chlore, un atome de brome, un groupe alkyle en C₁-C₄ (qui porte éventuellement un ou plusieurs substituants identiques ou différents fluoro et/ou bromo), un groupe alcoxy en C₁-C₄ (qui porte éventuellement un ou plusieurs substituants identiques ou différents fluoro et/ou chloro), un groupe alkyl(en C₁-C₄)thio, un groupe alkyl(en C₁-C₄)sulfinyle ou un groupe alkyl(en C₁-C₄)sulfonyle (qui portent éventuellement un ou plusieurs substituants identiques ou différents fluoro et/ou chloro), ou représentent un groupe di-(alkyl en C₁-C₄)aminosulfonyle;
en outre,
R³ représente le radical 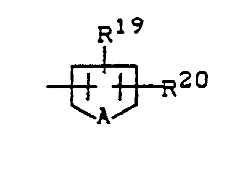 dans lequel
R¹⁹ et R²⁰ sont identiques ou différents et représentent un atome d'hydrogène, un atome de fluor, un atome de chlore, un atome de brome, un groupe cyano, un groupe nitro, un groupe alkyle en C₁-C₄ (qui porte éventuellement un ou plusieurs substituants identiques ou différents fluoro et/ou chloro), un groupe alcoxy en C₁-C₄ (qui porte éventuellement un ou plusieurs substituants identiques ou différents fluoro et/ou chloro), un groupe alkyl(en C₁-C₄)thio, un groupe alkyl(en C₁-C₄)sulfinyle ou un groupe alkyl(en C₁-C₄)sulfonyle (qui portent éventuellement un ou plusieurs substituants identiques ou différents fluoro et/ou chloro), un groupe di-(alkyl en C₁-C₄)aminosulfonyle, un groupe alcoxy(en C₁-C₄)carbonyle ou un groupe diméthylaminocarbonyle, et
A représente un atome d'oxygène, un atome de soufre ou encore le groupement N-Z¹ où
Z¹ représente un atome d'hydrogène; un groupe alkyle en C₁-C₄ (qui porte éventuellement un ou plusieurs substituants identiques ou différents fluoro, chloro, bromo ou cyano); un groupe cycloalkyle en C₃-C₆; un groupe benzyle; un groupe phényle (qui porte éventuellement un ou plusieurs substituants identiques ou différents fluoro, chloro, bromo ou nitro), un groupe alkyl(en C₁-C₄)carbonyle, un groupe alcoxy(en C₁-C₄)carbonyle ou un groupe di(alkyl en C₁-C₄)aminocarbonyle;
en outre,
R³ représente le radical 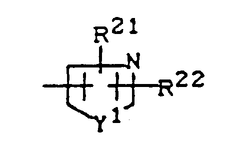 dans lequel
R²¹ et R²² sont identiques ou différents et représentent un atome d'hydrogène, un groupe alkyle en C₁-C₄, un atome d'halogène, un groupe alcoxy(en C₁-C₄)carbonyle, un groupe alcoxy en C₁-C₄ ou un groupe halogénalcoxy en C₁-C₄,
Y¹ représente un atome de soufre ou le groupement N-R²³ où
R²³ représente un atome d'hydrogène ou un groupe alkyle en C₁-C₄;
en outre,
R³ représente le radical 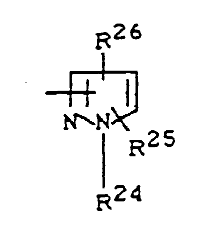 dans lequel
R²⁴ représente un atome d'hydrogène, un groupe alkyle en C₁-C₄, un groupe benzyle, un groupe pyridyle, un groupe quinolinyle ou un groupe phényle,
R²⁵ représente un atome d'hydrogène, un atome d'halogène, un groupe cyano, un groupe nitro, un groupe alkyle en C₁-C₄ (qui porte éventuellement un ou plusieurs substituants identiques ou différents fluoro et/ou chloro), un groupe alcoxy en C₁-C₄ (qui porte éventuellement un ou plusieurs substituants identiques ou différents fluoro et/ou chloro), un groupe dioxolanyle ou un groupe alcoxy(en C₁-C₄)carbonyle, et
R²⁶ représente un atome d'hydrogène, un atome d'halogène ou un groupe alkyle en C₁-C₄,
en outre
R³ représente un des groupements indiqués ci-après, 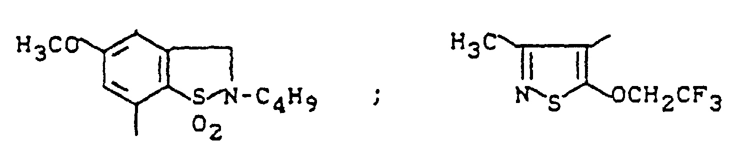 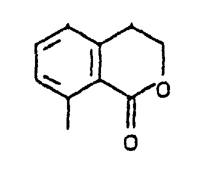 ainsi que des sels des composés de formule (I).

2. Sulfonylaminocarbonyltriazolinones portant des substituants liés via un atome de soufre, répondant à la formule (I) selon la revendication 1, dans lesquelles
n représente les nombres 0, 1 ou 2,
R¹ représente un groupe méthyle portant éventuellement un ou plusieurs substituants identiques ou différents fluoro; un groupe allyle; un groupe cyclopropyle; un groupe alcoxy en C₁-C₄; ou un groupe alcényl(en C₃-C₄)oxy,
R² représente un groupe alkyle en C₁-C₄ portant éventuellement un ou plusieurs substituants identiques ou différents fluoro, chloro, méthoxy ou éthoxy; un groupe alcényle en C₃-C₄ portant éventuellement un ou plusieurs substituants identiques ou différents fluoro et/ou chloro; ou représente un groupe cycloalkyle en C₃-C₆,
avec cette mesure que R² ne représente pas un groupe méthyle ou un groupe éthyle respectivement non substitués lorsque R¹ représente un groupe méthyle non substitué et n représente 0, et
R³ représente le groupement 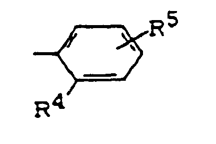 dans lequel
R⁴ représente un atome de fluor, un atome de chlore, un atome de brome, un groupe méthyle, un groupe trifluorométhyle, un groupe méthoxy, un groupe difluorométhoxy, un groupe trifluorométhoxy, un groupe 2-chloroéthoxy, un groupe 2-méthoxyéthoxy, un groupe alkyl(en C₁-C₃)thio, un groupe alkyl(en C₁-C₃)-sulfinyle, un groupe alkyl(en C₁-C₃)-sulfonyle, un groupe diméthylaminosulfonyle, un groupe diéthylaminosulfonyle, un groupe N-méthoxy-N-méthylaminosulfonyle, un groupe méthoxyaminosulfonyle, un groupe phényle, un groupe phénoxy ou un groupe alcoxy(en C₁-C₃)carbonyle, et
R⁵ représente un atome d'hydrogène, un atome de fluor, un atome de chlore ou un atome de brome;
en outre,
R³ représente le radical 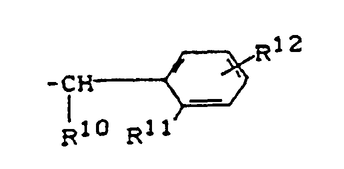 dans lequel
R¹⁰ représente un atome d'hydrogène,
R¹¹ représente un atome de fluor, un atome de chlore, un atome de brome, un groupe méthyle, un groupe méthoxy, un groupe difluorométhoxy, un groupe trifluorométhoxy, un groupe éthoxy, un groupe méthoxycarbonyle, un groupe éthoxycarbonyle, un groupe méthylsulfonyle ou un groupe diméthylaminosulfonyle, et
R¹² représente un atome d'hydrogène;
en outre,
R³ représente le radical 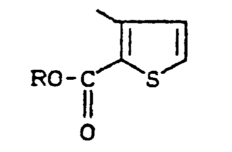 dans lequel R représente un groupe alkyle en C₁-C₄ ou bien
R³ représente le radical 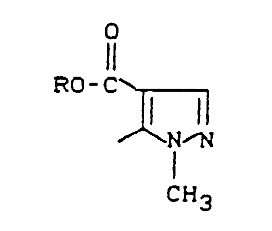 dans lequel R représente un groupe alkyle en C₁-C₄,
ainsi que des sels des composés de formule (I).

3. Procédé pour la préparation de sulfonylaminocarbonyltriazolinones portant des substituants liés via un atome de soufre, répondant à la formule générale (I) 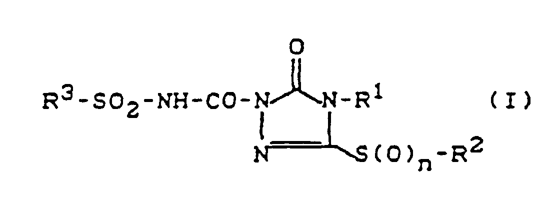 dans laquelle
n représente les nombres 0, 1 ou 2,
R¹ représente un groupe méthyle portant éventuellement un ou plusieurs substituants identiques ou différents fluoro, chloro ou bromo; un groupe allyle portant éventuellement un ou plusieurs substituants identiques ou différents fluoro, chloro et/ou bromo; un groupe cyclopropyle portant éventuellement un ou plusieurs substituants identiques ou différents fluoro, chloro, bromo et/ou méthyle; un groupe alcoxy en C₁-C₆ portant éventuellement un ou plusieurs substituants identiques ou différents fluoro, chloro ou cyano; ou encore un groupe alcényl(en C₃-C₄)oxy,
R² représente un groupe alkyle en C₁-C₆ portant éventuellement un ou plusieurs substituants identiques ou différents fluoro, chloro, cyano, cyclopropyle ou alcoxy en C₁-C₄; un groupe alcényle en C₃-C₆ ou un groupe alcynyle en C₃-C₆ portant respectivement le cas échéant un ou plusieurs substituants identiques ou différents fluoro, chloro et/ou bromo; ou représente un groupe cycloalkyle en C₃-C₆ portant éventuellement un ou plusieurs substituants identiques ou différents fluoro, chloro, bromo et/ou alkyle en C₁-C₄; avec cette mesure que R² ne représente pas un groupe méthyle ou un groupe éthyle respectivement non substitués lorsque R¹ représente un groupe méthyle non substitué et n représente 0, et
R³ représente le groupement 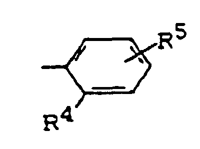 dans lequel
R⁴ et R⁵ sont identiques ou différents et représentent un atome d'hydrogène, un atome de fluor, un atome de chlore, un atome de brome, un atome d'iode, un groupe nitro, un groupe alkyle en C₁-C₆ (qui porte éventuellement un ou plusieurs substituants identiques ou différents fluoro, chloro, bromo, cyano, carboxyle, alcoxy(en C₁-C₄)carbonyle, alkyl(en C₁-C₄)aminocarbonyle, di-(alkyl en C₁-C₄)-aminocarbonyle, hydroxyle, alcoxy en C₁-C₄, formyloxy, alkyl(en C₁-C₄)-carbonyloxy, alcoxy(en C₁-C₄)carbonyloxy, alkyl(en C₁-C₄) aminocarbonyloxy, alkyl(en C₁-C₄)thio, alkyl(en C₁-C₄)sulfinyle, alkyl(en C₁-C₄)sulfonyle, di-(alkyl en C₁-C₄)aminosulfonyle, cycloalkyle en C₃-C₆ ou phényle); un groupe alcényle en C₂-C₆ (qui porte éventuellement un ou plusieurs substituants identiques ou différents fluoro, chlore, brome, cyano, alcoxy(en C₁-C₄)carbonyle, carboxyle ou phényle); un groupe alcynyle en C₂-C₆ (qui porte éventuellement un ou plusieurs substituants identiques ou différents fluoro, chloro, bromo, cyano, alcoxy(en C₁-C₄)carbonyle, carboxyle ou phényle); un groupe alcoxy en C₁-C₄ (qui porte éventuellement un ou plusieurs substituants identiques ou différents fluoro, chloro, bromo, cyano, carboxyle, alcoxy(en C₁-C₄)carbonyle, alcoxy en C₁-C₄, alkyl(en C₁-C₄)thio, alkyl(en C₁-C₄)-sulfinyle ou alkyl(en C₁-C₄)sulfonyle); un groupe alkyl(en C₁-C₄)thio (qui porte éventuellement un ou plusieurs substituants identiques ou différents fluoro, chlore, bromo, cyano, carboxyle, alcoxy(en C₁-C₄)carbonyle, alkyl(en C₁-C₄)thio, alkyl(en C₁-C₄)sulfinyle ou alkyl(en C₁-C₄)sulfonyle); un groupe alcényl(en C₃-C₆)oxy (qui porte éventuellement un ou plusieurs substituants identiques ou différents fluoro, chloro, bromo, cyano ou alcoxy(en C₁-C₄)carbonyle); un groupe alcényl(en C₂-C₆)thio (qui porte éventuellement un ou plusieurs substituants identiques ou différents fluoro, chloro, bromo, cyano, nitro, alkyl(en C₁-C₃)thio ou alcoxy(en C₁-C₄)carbonyle); un groupe alcynyl(en C₃-C₆) oxy; un groupe alcynyl(en C₃-C₆)thio, ou encore représente le radical -S(O)ₚ-R⁶ où
p représente les nombres 1 ou 2, et
R⁶ représente un groupe alkyle en C₁-C₄ (qui porte éventuellement un ou plusieurs substituants identiques ou différents fluoro, chloro, bromo, cyano ou alcoxy(en C₁-C₄)carbonyle); un groupe alcényle en C₃-C₆, un groupe alcynyle en C₃-C₆, un groupe alcoxy en C₁-C₄, un groupe alcoxy(en C₁-C₄)alkyl (en C₁-C₄)amine, un groupe alkyl(en C₁-C₄)amine, un groupe di-(alkyl en C₁-C₄)amine, un groupe phényle ou encore représente le radical -NHOR⁷ où
R⁷ représente un groupe alkyle en C₁-C₁₂ (qui porte éventuellement un ou plusieurs substituants identiques ou différents fluoro, chloro, cyano, alcoxy en C₁-C₄, alkyl(en C₁-C₄)thio, alkyl(en C₁-C₄)sulfinyle, alkyl(en C₁-C₄)sulfonyle, alkyl(en C₁-C₄)-carbonyle, alcoxy(en C₁-C₄)carbonyle, alkyl(en C₁-C₄)-aminocarbonyle ou di-(alkyl en C₁-C₄)aminocarbonyle); un groupe alcényle en C₃-C₆ (qui porte éventuellement un ou plusieurs substituants identiques ou différents fluoro, chloro ou bromo); un groupe alcynyle en C₃-C₆; un groupe cycloalkyle en C₃-C₆; un groupe cycloalkyl(en C₃-C₆)-alkyle en C₁-C₂; un groupe phénylalkyle en C₁-C₂ (qui porte éventuellement un ou plusieurs substituants identiques ou différents fluoro, chloro, nitro, cyano, alkyle en C₁-C₄, alcoxy en C₁-C₄ ou alcoxy(en C₁-C₄)-carbonyle); un groupe benzhydryle; ou encore un groupe phényle (qui porte éventuellement un ou plusieurs substituants identiques ou différents fluoro, chloro, nitro, cyano, alkyle en C₁-C₄, trifluorométhyle, alcoxy en C₁-C₄, fluoroalcoxy en C₁-C₂, alkyl(en C₁-C₄)thio, trifluorométhylthio ou alcoxy(en C₁-C₄)carbonyle),
R⁴ et/ou R⁵ représentent, en outre, un groupe phényle ou un groupe phénoxy; un groupe alkyl(en C₁-C₄)carbonylamino, un groupe alcoxy(en C₁-C₄)carbonylamino, un groupe alkyl(en C₁-C₄)aminocarbonylamino, un groupe di-(alkyl en C₁-C₄)aminocarbonylamino; ou représentent le radical -CO-R⁸ où
R⁸ représente un groupe alkyle en C₁-C₆, un groupe alcoxy en C₁-C₆, un groupe cycloalcoxy en C₃-C₆, un groupe alcényl(en C₃-C₆)oxy, un groupe alkyl(en C₁-C₄)thio, un groupe alkyl(en C₁-C₄)amine, un groupe alcoxy(en C₁-C₄)amine, un groupe alcoxy(en C₁-C₄)alkyl(en C₁-C₄)amino ou un groupe di-(alkyl en C₁-C₄)amine (qui portent éventuellement un ou plusieurs substituants identiques ou différents fluoro et/ou chloro),
R⁴ et/ou R⁵ représentent, en outre, un groupe triméthylsilyle, un groupe thiazolinyle, un groupe alkyl(en C₁-C₄)sulfonyloxy, un groupe di-(alkyl en C₁-C₄)aminosulfonylamino ou représentent le radical -CH=N-R⁹ où
R⁹ représente un groupe alkyle en C₁-C₆ portant éventuellement un ou plusieurs substituants identiques ou différents fluoro, chloro, cyano, carboxyle, alcoxy en C₁-C₄, alkyl(en C₁-C₄)thio, alkyl(en C₁-C₄)sulfinyle ou alkyl(en C₁-C₄)sulfonyle; un groupe benzyle portant éventuellement un ou plusieurs substituants identiques ou différents fluoro ou chloro; un groupe alcényle en C₃-C₆ ou un groupe alcynyle en C₃-C₆ portant éventuellement un ou plusieurs substituants identiques ou différents fluoro ou chloro; un groupe phényle portant éventuellement un ou plusieurs substituants identiques ou différents fluoro, chloro, bromo, alkyle en C₁-C₄, alcoxy en C₁-C₄, trifluorométhyle, trifluorométhoxy ou trifluorométhylthio; un groupe alcoxy en C₁-C₆, un groupe alcénoxy en C₃-C₆, un groupe alcynoxy en C₃-C₆ ou un groupe benzyloxy, chacun de ces groupes portant éventuellement un ou plusieurs substituants identiques ou différents fluoro et/ou chloro; un groupe amino, un groupe alkyl(en C₁-C₄)amino, un groupe di-(alkyl en C₁-C₄)amino, un groupe phénylamino, un groupe alkyl(en C₁-C₄)carbonylamino, un groupe alcoxy(en C₁-C₄)carbonylamino, un groupe alkyl(en C₁-C₄)sulfonylamino; ou représente un groupe phénylsulfonylamino portant éventuellement un ou plusieurs substituants identiques ou différents fluoro, chloro, bromo ou méthyle,
en outre,
R³ représente le radical 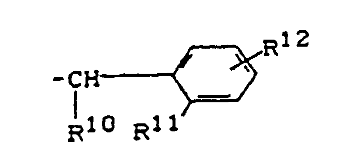 dans lequel
R¹⁰ représente un atome d'hydrogène ou un groupe alkyle en C₁-C₄,
R¹¹ et R¹² sont identiques ou différents et représentent un atome d'hydrogène, un atome de fluor, un atome de chlore, un atome de brome, un groupe nitro, un groupe cyano, un groupe alkyle en C₁-C₄ (qui porte éventuellement un ou plusieurs substituants identiques ou différents fluoro et/ou chloro); un groupe alcoxy en C₁-C₄ (qui porte éventuellement un ou plusieurs substituants identiques ou différents fluoro et/ou chloro); un groupe carboxyle, un groupe alcoxy(en C₁-C₄)carbonyle, un groupe diméthylaminocarbonyle, un groupe alkyl(en C₁-C₄)sulfonyle ou un groupe di-(alkyl en C₁-C₄)aminosulfonyle;
en outre,
R³ représente le radical 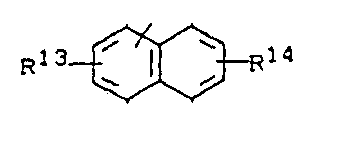 dans lequel
R¹³ et R¹⁴ sont identiques ou différents et représentent un atome d'hydrogène, un atome de fluor, un atome de chlore, un atome de brome, un groupe nitro, un groupe cyano, un groupe alkyle en C₁-C₄ (qui porte éventuellement un ou plusieurs substituants identiques ou différents fluoro et/ou chloro) ou un groupe alcoxy en C₁-C₄ (qui porte éventuellement un ou plusieurs substituants identiques ou différents fluoro et/ou chloro);
en outre,
R³ représente le radical 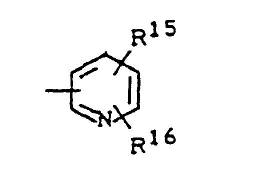 dans lequel
R¹⁵ et R¹⁶ sont identiques ou différents et représentent un atome d'hydrogène, un atome de fluor, un atome de chlore, un atome de brome, un groupe nitro, un groupe cyano, un groupe alkyle en C₁-C₄ (qui porte éventuellement un ou plusieurs substituants identiques ou différents fluoro et/ou chloro), un groupe alcoxy en C₁-C₄ (qui porte éventuellement un ou plusieurs substituants identiques ou différents fluoro et/ou chloro); un groupe alkyl(en C₁-C₄)thio, un groupe alkyl(en C₁-C₄)sulfinyle ou un groupe alkyl(en C₁-C₄)sulfonyle (qui portent éventuellement un ou plusieurs substituants identiques ou différents fluoro et/ou chloro); un groupe aminosulfonyle, un groupe mono(alkyl en C₁-C₄)aminosulfonyle; un groupe di-(alkyl en C₁-C₄)aminosulfonyle ou un groupe alcoxy(en C₁-C₄)carbonyle ou encore un groupe diméthylaminocarbonyle;
en outre,
R³ représente le radical 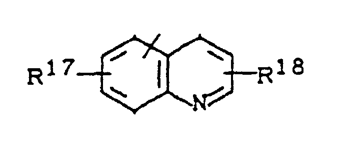 dans lequel
R¹⁷ et R¹⁸ sont identiques ou différents et représentent un atome d'hydrogène, un atome de fluor, un atome de chlore, un atome de brome, un groupe alkyle en C₁-C₄ (qui porte éventuellement un ou plusieurs substituants identiques ou différents fluoro et/ou bromo), un groupe alcoxy en C₁-C₄ (qui porte éventuellement un ou plusieurs substituants identiques ou différents fluoro et/ou chloro), un groupe alkyl(en C₁-C₄)thio, un groupe alkyl(en C₁-C₄)sulfinyle ou un groupe alkyl(en C₁-C₄)sulfonyle (qui portent éventuellement un ou plusieurs substituants identiques ou différents fluoro et/ou chloro), ou représentent un groupe di-(alkyl en C₁-C₄)aminosulfonyle;
en outre,
R³ représente le radical 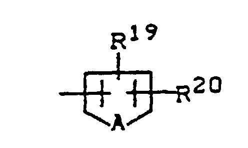 dans lequel
R¹⁹ et R²⁰ sont identiques ou différents et représentent un atome d'hydrogène, un atome de fluor, un atome de chlore, un atome de brome, un groupe cyano, un groupe nitro, un groupe alkyle en C₁-C₄ (qui porte éventuellement un ou plusieurs substituants identiques ou différents fluoro et/ou chloro), un groupe alcoxy en C₁-C₄ (qui porte éventuellement un ou plusieurs substituants identiques ou différents fluoro et/ou chloro), un groupe alkyl(en C₁-C₄)thio, un groupe alkyl(en C₁-C₄)sulfinyle ou un groupe alkyl(en C₁-C₄)sulfonyle (qui portent éventuellement un ou plusieurs substituants identiques ou différents fluoro et/ou chloro), un groupe di-(alkyl en C₁-C₄)aminosulfonyle, un groupe alcoxy(en C₁-C₄)carbonyle ou un groupe diméthylaminocarbonyle, et
A représente un atome d'oxygène, un atome de soufre ou encore le groupement N-Z¹ où
Z¹ représente un atome d'hydrogène; un groupe alkyle en C₁-C₄ (qui porte éventuellement un ou plusieurs substituants identiques ou différents fluoro, chloro, bromo ou cyano); un groupe cycloalkyle en C₃-C₆; un groupe benzyle; un groupe phényle (qui porte éventuellement un ou plusieurs substituants identiques ou différents fluoro, chloro, bromo ou nitro), un groupe alkyl(en C₁-C₄)carbonyle, un groupe alcoxy(en C₁-C₄)carbonyle ou un groupe di-(alkyl en C₁-C₄)aminocarbonyle;
en outre,
R³ représente le radical 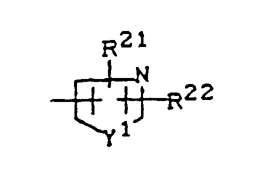 dans lequel
R²¹ et R²² sont identiques ou différents et représentent un atome d'hydrogène, un groupe alkyle en C₁-C₄, un atome d'halogène, un groupe alcoxy(en C₁-C₄)carbonyle, un groupe alcoxy en C₁-C₄ ou un groupe halogénalcoxy en C₁-C₄,
Y¹ représente un atome de soufre ou le groupement N-R²³ où
R²³ représente un atome d'hydrogène ou un groupe alkyle en C₁-C₄;
en outre,
R³ représente le radical 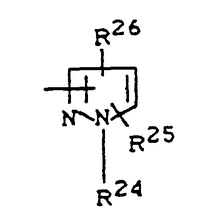 dans lequel
R²⁴ représente un atome d'hydrogène, un groupe alkyle en C₁-C₄, un groupe benzyle, un groupe pyridyle, un groupe quinolinyle ou un groupe phényle,
R²⁵ représente un atome d'hydrogène, un atome d'halogène, un groupe cyano, un groupe nitro, un groupe alkyle en C₁-C₄ (qui porte éventuellement un ou plusieurs substituants identiques ou différents fluoro et/ou chloro), un groupe alcoxy en C₁-C₄ (qui porte éventuellement un ou plusieurs substituants identiques ou différents fluoro et/ou chloro), un groupe dioxolanyle ou un groupe alcoxy(en C₁-C₄)carbonyle, et
R²⁶ représente un atome d'hydrogène, un atome d'halogène ou un groupe alkyle en C₁-C₄,
en outre
R³ représente un des groupements indiqués ci-après, 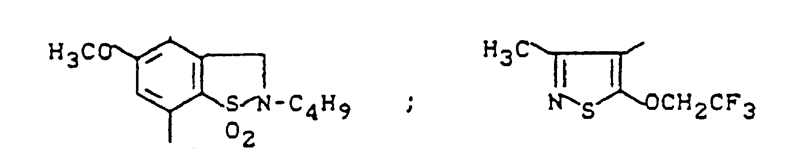 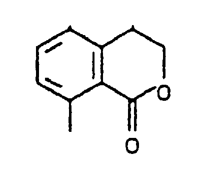 ainsi que des sels des composés de formule (I),
caractérisé en ce que
a) on fait réagir des triazolinones répondant à la formule générale (II) 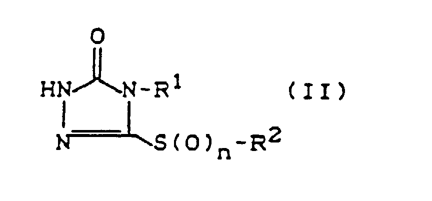 dans laquelle
n, R¹ et R² ont les significations indiquées ci-dessus (dans la formule I),
avec des sulfonylisocyanates répondant à la formule générale (III)
R³-SO₂-N=C=O (III)
dans laquelle
R³ a la signification indiquée ci-dessus,
éventuellement en présence d'un diluant, ou bien en ce que
b) on fait réagir des dérivés de triazolinones répondant à la formule générale (IV) 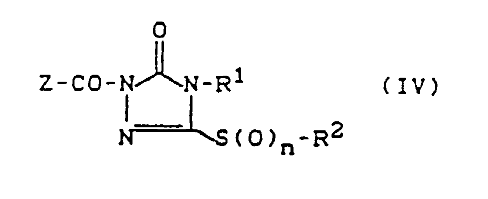 dans laquelle
n, R¹ et R² ont les significations indiquées ci-dessus et
Z représente un atome de chlore, un groupe alcoxy en C₁-C₄, un groupe benzyloxy ou un groupe phénoxy,
avec des amides d'acides sulfoniques répondant à la formule générale (V)
R³-SO₂-NH₂ (V)
dans laquelle
R³ a la signification indiquée ci-dessus,
éventuellement en présence d'un agent accepteur d'acide et éventuellement en présence d'un diluant, ou bien en ce que
c) on fait réagir des triazolinones répondant à la formule générale (II) 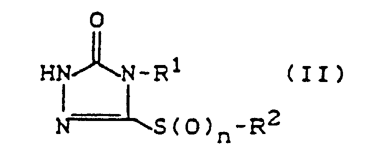 dans laquelle
n, R¹ et R² ont les significations indiquées ci-dessus,
avec des dérivés d'amides d'acides sulfoniques répondant à la formule générale (VI)
R³-SO₂-NH-CO-Z (VI)
dans laquelle
R³ a la signification indiquée ci-dessus et
Z représente un atome de chlore, un groupe alcoxy en C₁-C₄, un groupe benzyloxy ou un groupe phénoxy,
éventuellement en présence d'un agent accepteur d'acide et éventuellement en présence d'un diluant, et le cas échéant, à partir des composés de formule (I) préparés conformément aux procédés (a), (b) ou (c), on obtient des sels conformément à des procédés habituels.

4. Agents herbicides caractérisés par une teneur en au moins une sulfonylaminocarbonyltriazolinone de formule (I) selon la revendication 1 ou 3.

5. Utilisation de sulfonylaminocarbonyltriazolinones de formule (I) selon la revendication 1 ou 3 pour lutter contre la croissance non désirée des plantes.

6. Procédé pour lutter contre les mauvaises herbes, caractérisé en ce qu'on laisse agir des sulfonylaminocarbonyltriazolinones de formule (I) selon la revendication 1 ou 3 sur les mauvaises herbes ou sur leur biotope.

7. Procédé pour préparer des agents herbicides, caractérisé en ce qu'on mélange des sulfonylaminocarbonyltriazolinones de formule (I) selon la revendication 1 ou 3 avec des diluants et/ou des agents tensioactifs.
